(19) Europäisches Patentamt
European Patent Office
Office européen des brevets

(11) EP 1 325 730 B1

(12) # FASCICULE DE BREVET EUROPEEN

(45) Date de publication et mention de la délivrance du brevet:
**04.10.2006 Bulletin 2006/40**

(51) Int Cl.:
***A61K 8/04*** *(2006.01)* ***A61K 8/37*** *(2006.01)*
***A61Q 1/06*** *(2006.01)*

(21) Numéro de dépôt: **02293088.7**

(22) Date de dépôt: **13.12.2002**

(54) **Composition de maquillage ou de soin des matières kératiniques comprenant une huile hydrocarbonée non volatile, une phase particulaire et un agent dispersant particulier**

Zusammensetzung zum Schminken oder zur Pflege von Keratinfasern enthaltend ein nichtflüchtiges Kohlenwasserstofföl, eine Partikelphase und ein Dispergiermittel

Composition for make-up or care of keratinic fibers comprising a non-volatile hydrocarbon oil, a particulate phase and a specific dispersing agent

(84) Etats contractants désignés:
**AT BE BG CH CY CZ DE DK EE ES FI FR GB GR IE IT LI LU MC NL PT SE SI SK TR**

(30) Priorité: **08.01.2002 FR 0200161**

(43) Date de publication de la demande:
**09.07.2003 Bulletin 2003/28**

(73) Titulaire: **L'Oreal-D.I.P.I.**
**92600 Asnieres (FR)**

(72) Inventeurs:
- **Arnaud, Pascal**
**94240 L'Hay Les Roses (FR)**
- **Filippi, Vanina**
**75015 Paris (FR)**
- **Blin, Xavier**
**75015 Paris (FR)**

(74) Mandataire: **Le Coupanec, Pascale A.M.P.**
**Nony & Associés**
**3, rue de Penthièvre**
**75008 Paris (FR)**

(56) Documents cités:
**EP-A- 0 559 320**
**EP-A- 1 184 028**
**WO-A-00/56280**
**WO-A-00/74640**
**WO-A-93/23483**
**WO-A-97/16157**
**US-A- 6 132 750**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à une composition cosmétique anhydre contenant un agent dispersant particulier. Cette composition possède des propriétés cosmétiques remarquables, en particulier de tenue, et confère au maquillage ou au soin des propriétés de brillance, de confort et de non-migration.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eye liners, les mascaras, les compositions de protection solaire, de coloration ou de bronzage artificiel de la peau ou encore de maquillage du corps ou des cheveux.

Les produits de maquillage ou de soin de la peau ou des lèvres des êtres humains comme les fonds de teint ou les rouges à lèvres contiennent généralement des phases grasses telles que des cires et des huiles, des pigments et/ou charges et éventuellement des additifs comme des actifs cosmétiques. Elles peuvent aussi contenir des produits dits "pâteux", de consistance souple, permettant d'obtenir des pâtes, colorées ou non, à appliquer au pinceau.

Les compositions, notamment de maquillage, connues ont tendance à migrer, c'est-à-dire à se propager au cours du temps à l'intérieur des plis des rides et des ridules de la peau qui entourent notamment les lèvres et les yeux, entraînant un effet inesthétique. Cette migration est souvent citée par les femmes comme un défaut majeur des rouges à lèvres et des fards à paupières classiques. Par "migration", on entend un débordement de la composition et en particulier de la couleur, hors du tracé initial du maquillage.

En outre, ces compositions présentent une mauvaise tenue dans le temps et en particulier de la couleur. Cette mauvaise tenue se caractérise par une modification de la couleur (virage, palissement) généralement par suite d'une interaction avec le sébum et/ou la sueur sécrétés par la peau dans le cas de fond de teint et de fard à joues ou à paupières, ou d'une interaction avec la salive dans le cas des rouges à lèvres. De plus, cette modification de couleur est souvent non homogène. Ceci oblige l'utilisateur à se remaquiller très souvent, ce qui peut constituer une perte de temps.

Les problèmes de tenue de la couleur étaient jusqu'ici résolus en introduisant un taux élevé de pigments dans les compositions, de manière à ce que celles ci déposent, sur le support sur lequel elles sont appliquées, une quantité importante de pigments.

Il a également été proposé des compositions contenant des composés volatils, qui, bien que présentant des propriétés de tenue améliorées, ont l'inconvénient de laisser sur la peau et les lèvres, après évaporation des composés volatils, un film qui devient inconfortable au cours du temps (sensation de dessèchement et de tiraillement), écartant un certain nombre de femmes de ce type de rouge à lèvres.
En outre, ces compositions conduisent à des films colorés mats et couvrants. Or, les femmes sont aujourd'hui à la recherche de produits, notamment de coloration des lèvres ou des paupières, brillants et semi-couvrants.

Il est connu par ailleurs que l'amélioration des propriétés de brillance nécessite une bonne dispersion des particules solides dans la composition, en particulier des pigments.

Le brevet US-A-5 945 092 de Revlon décrit ainsi l'utilisation de tensioactifs siliconés associés avec des huiles volatiles et des agents dispersants fluorés. Cependant, ces tensioactifs présentent l'inconvénient d'être potentiellement irritants notamment pour la muqueuse labiale lorsque leur pourcentage dans la composition est important (typiquement supérieur à 3 %), et ceci d'autant plus que le taux d'huile volatile est élevé (supérieur à 30 % typiquement). Les agents dispersants fluorés décrits dans les exemples de ce brevet n'ont pas les paramètres de solubilité tels que définis selon la présente invention. EP- 1112734 décrit des compositions cosmétiques contenant un agent dispersant moins plus de 10% d'huile volatile.

La société Kao a proposé dans sa demande EP-A-0548694 une composition contenant un tensioactif siliconé (silicone modifiée polyéther), des huiles et des pigments, ayant un bon confort à l'utilisation et une tenue améliorée. Toutefois, ces compositions ne permettent pas d'obtenir un maquillage ayant une tenue suffisante.
De plus, on privilégie actuellement, dans le domaine de la cosmétique, l'utilisation de composés d'origine naturelle. Or les tensio actifs siliconés et fluorés prévus dans les compositions des documents ci-dessus sont d'origine synthétique.

Il subsiste donc le besoin d'une composition ne présentant pas les inconvénients ci-dessus et ayant notamment de bonnes propriétés de tenue, qui migre peu ou pas, tout en conférant au dépôt de maquillage ou de soin un aspect plus ou moins brillant, adapté au désir de la consommatrice, ne desséchant pas et ne tiraillant pas la peau ou les lèvres sur lesquelles elle est appliquée, aussi bien lors de l'application qu'au cours du temps, et n'irritant pas la peau ou les lèvres.

Le demandeur a constaté, de façon surprenante, que l'utilisation de l'association d'une huile hydrocarbonée non volatile, d'une phase particulaire inerte et d'un agent dispersant hydrocarboné permettait l'obtention d'une composition de bonne tenue, notamment en couleur, brillante, confortable et qui migre peu ou pas, et non irritante.

La présente invention a donc pour objet une composition anhydre de soin ou de maquillage des matières kératiniques, comprenant un milieu cosmétiquement acceptable contenant

- au moins une huile hydrocarbonée non volatile,
- au moins une phase particulaire contenant au moins une charge inerte et
- au moins un agent dispersant hydrocarboné comportant des atomes de carbone et d'hydrogène et une ou plusieurs fonctions choisies parmi les fonctions hydroxyle, ester, éther, carboxylique, amide, ledit agent dispersant présentant les paramètres de solubilité δd et δa satisfaisant aux conditions suivantes : $16,2 \leq \delta_d \leq 20\ (J/cm^3)^{1/2}$ et $9,1 \leq \delta_a \leq 20\ (J/cm^3)^{1/2}$.

Par « au moins » un composé, on entend un ou plusieurs composés.

Par « composition cosmétique anhydre », on entend une composition comprenant une phase continue (appelée aussi phase externe) grasse qui représente jusqu'à 95 % en poids de la composition, de préférence jusqu'à 98% en poids, de préférence encore jusqu'à 99,5 % en poids.

L'invention a également pour objet un procédé cosmétique pour conférer à un film de composition cosmétique anhydre des propriétés de tenue, de brillant, de confort et/ou de non-migration, consistant à introduire dans ladite composition au moins une huile hydrocarbonée non volatile, au moins une phase particulaire inerte et au moins un agent dispersant hydrocarboné qui présente les paramètres de solubilité δd et δa satisfaisant aux conditions suivantes : $16,2 \leq \delta_d \leq 20\ (J/cm^3)^{1/2}$ et $9,1 \leq \delta_a \leq 20\ (J/cm^3)^{1/2}$.

L'invention a encore pour objet l'utilisation de l'association d'au moins une huile hydrocarbonée non volatile, d'au moins une phase particulaire inerte et d'au moins un agent dispersant hydrocarboné qui présente les paramètres de solubilité δd et δa satisfaisant aux conditions suivantes : $16,2 \leq \delta_d \leq 20\ (J/cm^3)^{1/2}$ et $9,1 \leq \delta_a \leq 20\ (J/cm^3)^{1/2}$, dans une composition cosmétique anhydre de bonne tenue, brillante, confortable et /ou non migrante.

L'invention a enfin pour objet l'utilisation de l'association d'au moins une huile hydrocarbonée non volatile, d'au moins une phase particulaire inerte et d'au moins un agent dispersant hydrocarboné qui présente les paramètres de solubilité δd et δa satisfaisant aux conditions suivantes : $16,2 \leq \delta_d \leq 20\ (J/cm^3)^{1/2}$ et $9,1 \leq \delta_a \leq 20\ (J/cm^3)^{1/2}$, dans une composition cosmétique anhydre, comme agent pour conférer à ladite composition des propriétés de tenue, de brillant, de confort et/ou de non-migration.

L'agent dispersant hydrocarboné et l'huile hydrocarbonée non volatile de la composition selon l'invention sont des composés distincts.

Par « matières kératiniques », on entend la peau, les lèvres et les phanères.

Par composé "non-volatil", on entend un composé susceptible de rester sur la peau ou les lèvres pendant plusieurs heures. Un composé non-volatil a en particulier une pression de vapeur, à température ambiante et pression atmosphérique, non nulle, inférieure à 0,02 mm de Hg (2,66 Pa).

Par composé "volatil", on entend un composé susceptible de s'évaporer de la peau ou des lèvres, en moins d'une heure. Un composé volatil est notamment choisi parmi les composés ayant une pression de vapeur, à température ambiante et pression atmosphérique allant de 0,02 mm à 300mm de Hg (2,66 Pa à 40 000 Pa) et mieux allant de 0,1 à 90 mm de Hg (13 Pa à 12 000 Pa).

Par "huile", on entend tout milieu non aqueux liquide et insoluble dans l'eau à température ambiante (25°C) et pression atmosphérique (760mm de Hg ou $1,01.10^5$Pa).

Par "phase particulaire inerte", on entend toute charge solide à température ambiante et pression atmosphérique, utilisée seule ou en association, ne réagissant pas chimiquement avec les différents ingrédients de la composition et qui sont insolubles dans ces ingrédients, même lorsque ces ingrédients sont portés à une température supérieure à la température ambiante (température de fusion par exemple de ces ingrédients).

Cette composition contient des ingrédients compatibles avec les matières kératiniques, à savoir la peau, les lèvres, les fibres kératiniques et les ongles. Elle peut se présenter sous forme de gel anhydre. Elle peut se présenter, en outre, sous forme plus ou moins fluide, de pâte ou de solide non déformable ou rigide, éventuellement coulé en stick ou en coupelle. De préférence, elle se présente sous forme fluide ou de stick. Par "fluide", on entend une composition s'écoulant sous son propre poids, à l'inverse d'un solide.

La composition de l'invention contient peu ou pas d'huiles volatiles c'est à dire moins de 10 % par rapport au poids total de la composition, de préférence moins de 5 % et mieux moins de 2 % et avantageusement est exempte d'huile volatile.

L'agent dispersant hydrocarboné utilisé dans la composition selon l'invention sert à protéger les particules dispersées contre leur agglomération ou floculation. Par composé « hydrocarboné », on entend un composé comportant des atomes de carbone et d'hydrogène et une ou plusieurs fonctions choisies parmi les fonctions hydroxyle, ester, éther, carboxylique, amide. L'agent dispersant hydrocarboné de l'invention est dépourvu d'atomes de fluor. Cet agent porte une ou des fonctionnalités ayant une affinité forte pour la surface des particules à disperser.

De préférence, l'agent dispersant hydrocarboné (appelé par la suite «dispersant hydrocarboné»), de la composition selon l'invention, est fluide à température ambiante (25°C), et notamment liquide et/ou présente un indice de réfraction ≥ 1,45 à 20°C (l'indice de réfraction étant mesuré au réfractomètre).

Ce dispersant hydrocarboné présente les paramètres de solubilité δd et δa selon l'espace de solubilité de

Hansen satisfaisant aux conditions suivantes :

$16,2 \leq \delta_d \leq 20\ (J/cm^3)^{1/2}$ de préférence $16,3 \leq \delta_d \leq 19\ (J/cm^3)^{1/2}$, et mieux $16,9 \leq \delta_d \leq 18\ (J/cm^3)^{1/2}$. et
$9,1 \leq \delta_a \leq 20\ (J/cm^3)^{1/2}$, de préférence $10 \leq \delta_a \leq 18,1\ (J/cm^3)^{1/2}$, et mieux $13 \leq \delta_a \leq 14,5\ (J/cm^3)^{1/2}$

La définition des paramètres de solubilité selon HANSEN est bien connue de l'homme du métier, et notamment décrite dans l'article de C. M. HANSEN : "The three dimensional solubility parameters" J. Paint Technol. 39, 105 (1967). Ces paramètres sont aussi décrits dans le document JP-A-08-109121 de KAO et le document de D.W. Van KREVELEN "Properties of polymers" (1990), p. 190 °

Selon cet espace de HANSEN :

- $\delta_d$ caractérise les forces de dispersion de LONDON issues de la formation de dipôles induits lors des chocs moléculaires ;
- $\delta_P$ caractérise les forces d'interactions de DEBYE entre dipôles permanents ;
- $\delta_h$ caractérise les forces d'interactions spécifiques (type liaisons hydrogène, acide/base, donneur/accepteur, etc.) ;

Les paramètres $\delta_d$, $\delta_P$ et $\delta_h$ sont généralement exprimés en $(J/cm^3)^{1/2}$. Ils sont déterminés à température ambiante (25°C) et en particulier selon la méthode de calcul indiquée dans le document brevet de KAO ci-dessus.

Dans la composition selon l'invention, on peut utiliser n'importe quel dispersant hydrocarboné fluide, et en particulier liquide, ou mélange de dispersants hydrocarbonés fluides satisfaisant aux relations ci-dessus. Dans ce cas, les paramètres de solubilité du mélange sont déterminés à partir de ceux des dispersants hydrocarbonés fluides pris séparément, selon les relations suivantes :

$$\delta_{Dmel} = \sum_i xi\, \delta_{Di}\ ; \qquad \delta_{pmel} = \sum_i xi\, \delta_{pi} \quad \text{et} \quad \delta_{hmel} = \sum_i xi\, \delta_{hi}$$

où xi représente la fraction volumique du dispersant hydrocarboné fluide (i) dans le mélange.

Il est à la portée de l'homme du métier de déterminer les quantités de chaque dispersant hydrocarboné fluide pour obtenir un mélange de dispersants hydrocarbonés fluides satisfaisant aux relations ci-dessus.

Avantageusement, le dispersant hydrocarboné présente une structure chimique comportant au moins un groupement polaire choisi parmi -COOH ; -OH ; oxyde d'éthylène : $-(O-CH_2-CH_2-)$ ;oxyde de propylène

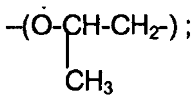

$-PO_4$ ; NHR ; $NR_1R_2$ avec $R_1$, $R_2$ formant éventuellement un cycle et représentant un radical alkyle ou alkoxy linéaire ou ramifié en $C_1$ à $C_{20}$ ou

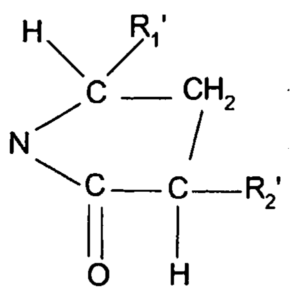

avec $R_1'$ et $R_2'$ qui peuvent être égal à H ou à une chaîne alkyle ou alkoxy linéaire ou ramifiée en $C_1$ à $C_{20}$.

Le dispersant hydrocarboné selon l'invention peut être choisi parmi :

- les alcools gras modifiés éther et en particulier les produits d'addition de l'oxyde d'éthylène et / ou de l'oxyde de

propylène avec i) un alcool gras linéaire ou ramifié ou avec ii) un alkylphénol,

- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et / ou l'huile de ricin hydrogénée,
- les phosphate trialkylés et les mono, di et triphosphate alkylés, et leurs mélanges,

ces composés satisfaisant aux paramètres de solubilité définis ci-dessus.

Le mot ester selon l'invention signifie un monoester, un diester, un triester et plus généralement un polyester.

De préférence, le dispersant hydrocarboné est choisi parmi les monoesters, les diesters et les esters résultant d'une estérification partielle, c'est à dire que l'ester final comporte une ou plusieurs fonctions -OH libre.

Avantageusement, le dispersant hydrocarboné est choisi parmi :

- les produits d'addition de 2 à 30 moles d'oxyde d'éthylène et / ou de 0 à 5 moles d'oxyde de propylène avec i) un alcool gras linéaire ou ramifié en $C_8$ à $C_{40}$, et mieux en $C_8$ à $C_{22}$ ou avec ii) un alkylphénol,
- les esters résultant de la réaction d'au moins un acide gras en $C_8$ à $C_{40}$, et mieux en $C_8$ à $C_{22}$, avec au moins un produit d'addition de 1 à 30 moles d'oxyde d'éthylène et du glycérol ou avec au moins un produit d'addition de 1 à 30 moles d'oxyde d'éthylène et du polyglycérol,
- les esters résultant de la réaction du glycérol ou du polyglycérol avec au moins un produit d'addition de 2 à 30 moles d'oxyde d'éthylène et d'un acide gras saturé ou insaturé en $C_8$ à $C_{40}$, et mieux en $C_8$ à $C_{22}$,
- les esters partiels résultant de la réaction d'au moins un acide gras linéaire ou ramifié, saturé ou insaturé en $C_8$ à $C_{40}$ et mieux en $C_8$ à $C_{22}$, d'acide ricinoléique ou d'acide 12-hydroxystéarique, avec le glycérol, le polyglycérol, le pentaérythritol ou le sorbitol,
- les esters résultant de la réaction du sorbitan avec au moins un acide gras linéaire ou ramifié, saturé ou insaturé en $C_8$ à $C_{40}$ et mieux en $C_8$ à $C_{22}$,
- les esters résultant iii) de la réaction du sorbitan avec au moins un produit d'addition de 2 à 30 moles d'oxyde d'éthylène et d'un acide gras saturé ou insaturé en $C_8$ à $C_{40}$ et mieux en $C_8$ à $C_{22}$ ou iv) de la réaction d'au moins un acide gras saturé ou insaturé en $C_8$ à $C_{40}$ et mieux en $C_8$ à $C_{22}$, avec au moins un produit d'addition de 2 à 30 moles d'oxyde d'éthylène et du sorbitan,
- les produits d'addition de 2 à 60 moles d'oxyde d'éthylène avec l'huile de ricin et / ou l'huile de ricin hydrogénée,
- les phosphate trialkylés et les mono, di et triphosphate alkylés,
- et leurs mélanges.

De manière préférentielle, le dispersant hydrocarboné est choisi parmi :
l'alcool de myristyle oxyéthyléné à 15 groupements oxyde d'éthylène (ou DE) ($\delta_d$ = 17,33 $(J/cm^3)^{1/2}$ et $\delta_a$ = 9,28 $(J/cm^3)^{1/2}$), le monoisostéarate de polyglycérol-2 oxyéthyléné à 5 OE ($\delta_d$ = 17,34 $(J/cm^3)^{1/2}$ et $\delta_a$ = 12,22 $(J/cm^3)^{1/2}$), le diisostéarate de polyglycérol-3 ($\delta_d$ = 16,96 $(J/cm^3)^{1/2}$ et $\delta_a$ = 10,4 $(J/cm^3)^{1/2}$), le monoisostéarate de glycérol ($\delta_d$ = 16,32 $(J/cm^3)^{1/2}$ et $\delta_a$ = 11,01 $(J/cm^3)^{1/2}$), le monoisostéarate de polyglycérol-2 ($\delta_d$ = 17,03 $(J/cm^3)^{1/2}$ et $\delta_a$ = 13,25 $(J/cm^3)^{1/2}$), l'isostéarate de polyglycérol-3 ($\delta_d$ = 17,38 $(J/cm^3)^{1/2}$ et $\delta_a$ = 14,48 $(J/cm^3)^{1/2}$), l'isostéarate de polyglycérol-4 ($\delta_d$ = 17,57 $(J/cm^3)^{1/2}$ et $\delta_a$ = 15,37 $(J/cm^3)^{1/2}$), le monoisostéarate de polyglycérol-6 ($\delta_d$ = 17,86 $(J/cm^3)^{1/2}$ et $\delta_a$ = 16,61 $(J/cm^3)^{1/2}$), le monoisostéarate de polyglycérol-10 ($\delta_d$ = 18,22 $(J/cm^3)^{1/2}$ et $\delta_a$ = 18,41 $(J/cm^3)^{1/2}$), le monooléate de polyglycérol-2 ($\delta_d$ = 17,14 $(J/cm^3)^{1/2}$ et $\delta_a$ = 13,39 $(J/cm^3)^{1/2}$), l'isostéarate de sorbitan ($\delta_d$= 17,33 $(J/cm^3)^{1/2}$ et $\delta_a$ = 13,56 $(J/cm^3)^{1/2}$), le monooléate de sorbitan ($\delta_d$ = 17,32 $(J/cm^3)^{1/2}$ et $\delta_a$ = 13,66 $(J/cm^3)^{1/2}$), le monooléate de sorbitan oxyéthyléné à 5 OE ($\delta_d$ = 17,56 $(J/cm^3)^{1/2}$ et $\delta_a$= 12,47 $(J/cm^3)^{1/2}$), et leurs mélanges.

Avantageusement, le dispersant hydrocarboné est choisi parmi les esters partiels de polyglycérol et d'acide isostéarique, les esters partiels de polyglycérol et d'acide oléique, les esters partiels de sorbitan et d'acide oléique, et leurs mélanges.

Comme dispersant hydrocarboné utilisable préférentiellement dans la composition selon l'invention, on peut choisir le monoisostéarate de polyglycérol-2 tel que le Salacos 41 fabriqué ou commercialisé par la société Nisshin Oil Mills, le diisostéarate de polyglycérol-3 tel que le Lameform TGI fabriqué ou commercialisé par la société Cognis, le

monooléate de polyglycérol-2 tel que le Rylo PG 29 fabriqué ou commercialisé par la société Danisco Ingredients, le monooléate de sorbitan tel que le Span 80 fabriqué ou commercialisé par la société Uniqema, et leurs mélanges.

Les quantités des différents ingrédients de la composition selon l'invention seront données en pourcentages en poids par rapport au poids total de la composition.

L'agent dispersant hydrocarboné représente de 0,5 à 40 %, de préférence de 3 à 20% et mieux de 5 à 15% du poids total de la composition.

L'huile hydrocarbonée non volatile utilisée dans la composition selon l'invention a avantageusement une masse molaire allant de 200 à 1500 g/mol, de préférence de 220 à 500 g/mol, et mieux de 230 à 430 g/mol.

Comme huile hydrocarbonée non volatile utilisable dans l'invention, on peut citer les esters sous forme monoester, diester et de façon générale polyester ramifié et saturé.

Ces esters sont de préférence choisis parmi les esters résultant de la réaction d'un acide carboxylique en $C_2$ à $C_{30}$, et mieux en $C_2$ à $C_{18}$ avec un alcool en $C_2$ à $C_{30}$, et mieux en $C_2$ à $C_{20}$ ou un polyol en $C_2$ à $C_{20}$ et mieux en $C_2$ à $C_8$, et leurs mélanges.

Comme huile hydrocarbonée non volatile utilisable dans l'invention, on peut citer :

- les esters de l'acide néopentanoïque comme l'isodecyl neopentanoate (242,4), l'isotridecyl neopentanoate (270,44), l'isostearyl neopentanoate (354,62), l'octyldodecyl neopentanoate (382,67) ;
- les esters de l'acide isononanoïque comme l'isononyl isononanoate (284,48), l'octyl isononanoate (270,44), l'isodecyl isononanoate (298,51), l'isotridecyl isononanoate (340,59), l'isostearyl isononanoate (410,73),
- les esters de l'alcool isopropylique, tels que l'isopropyl myristate (270,46), l'isopropyl palmitate (298,51), l'isopropyl stearate ou isostearate (326,56),
- et l'éthyl 2-hexyl palmitate (368,64), le $C_{12}$-$C_{15}$ alkyl benzoate (309,04), le neopentyl glycol diheptanoate (328,49), le propyleneglycol diethyl 2-hexanoate (328,5), l'ethyl hexyle ethylhexanoate (256,43),
- et leurs mélanges.

Ces esters sont cités en noms CTFA (International Cosmetic Ingredient Dictionary, 5ème édition et suivantes). Les chiffres entre parenthèses correspondent à leur masse molaire exprimée en g/Mol.

De préférence, l'huile hydrocarbonée non volatile est choisie parmi les esters de l'acide néopentanoïque les esters de l'acide isononanoïque, et leurs mélanges.

L'huile hydrocarbonée non volatile peut représenter de 5 à 98 %, de préférence de 7 à 60 %, mieux de 10 à 50 %, et encore mieux de 10 à 30 % du poids total de la composition.

La composition selon l'invention comprend également une phase particulaire inerte qui contient au moins une charge inerte absorbante ou non, c'est-à-dire une ou plusieurs charges inertes en particulier absorbant les huiles. De préférence, ces charges ont un diamètre apparent allant de 0,01 à 150 $\mu$m et mieux de 0,5 à 150 $\mu$m. Un diamètre apparent correspond au diamètre du cercle dans lequel s'inscrit la particule élémentaire selon sa plus petite dimension (épaisseur pour des lamelles).

Les charges inertes peuvent être choisies parmi les charges minérales ou organiques, lamellaires, sphériques ou oblongues. De préférence, la charge inerte est choisie parmi le talc, le mica, la silice, le kaolin, les poudres de polyamide comme le Nylon® (Orgasol® de chez Atochem), de poly-β-alanine et de polyéthylène, les poudres de polytétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), les copolymères d'acide acrylique (Polytrap® de Dow Corning) et les microbilles de résine de silicone (Tospearl® de Toshiba, par exemple), le carbonate de calcium précipité, le dicalcium phosphate, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

La phase particulaire inerte peut représenter de 0,1 à 30 % du poids total de la composition et mieux de 2 à 25 % et encore mieux de 3 à 20 %.

La composition de l'invention peut comprendre avantageusement au moins une matière colorante. Cette matière colorante contient au moins un composé pulvérulent et/ou un ou plusieurs colorants liposolubles ou hydrosolubles, par exemple à raison de 0 à 70% du poids total de la composition et notamment de 0,01 à 70%. Le ou les composés pulvérulents peuvent être choisis parmi les pigments, les nacres ou pigments nacrés, habituellement utilisés dans les compositions cosmétiques, et leurs mélanges.

Avantageusement, la matière colorante représente jusqu'à 50 % du poids total de la composition, par exemple de 0,001 à 50 % en poids de préférence de 0,01 à 40 %, et mieux de 0,05 à 30%.

Par « pigment », on entend des particules blanches ou colorées, minérales ou organiques, insolubles dans corps gras tels que les huiles, destinées à colorer et/ou opacifier la composition.

Par « nacres » ou « pigments nacrés », on entend des particules irisées, notamment produites par certains mollusques dans leur coquille ou bien synthétisées, insolubles dans les corps gras tels que les huiles.
Par « colorants », il faut comprendre des composés généralement organiques solubles dans les corps gras comme les huiles ou dans une phase hydroalcoolique.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres ou pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine, le rocou. Ils peuvent représenter de 0 à 20 % et notamment 0,01 à 20 % du poids de la composition et mieux de 0,1 à 6 %. Les colorants hydrosolubles sont par exemple le jus de betterave, le bleu de méthylène et peuvent représenter jusqu'à 6 % du poids total de la composition.

Avantageusement, la composition selon l'invention comprend un composé siliconé non volatil. Ce composé est de préférence choisi parmi les composés liquides à température ambiante et de manière encore plus préférentielle, ils ont une viscosité comprise dans la gamme allant de 5 à 100 000 cSt à 25°C et mieux de 10 à 50 000 cSt, de préférence de 10 à 5 000 cSt.

A titre d'exemples de composés siliconés, on peut citer les polydiméthylsiloxanes, les phényltriméthicones, les polyalkylméthylsiloxanes, les résines de silicones telles celles décrites dans les documents JP-A-62-61911, JP-A-61-65809 et EP-A-602905, les silicones fluorées et leurs mélanges.

En particulier, ces composés siliconés sont choisis parmi les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les silicones fluorées comportant un groupement fluoré pendant ou en bout de chaîne siliconée ayant de 1 à 12 atomes de carbone dont tout ou partie des hydrogènes est substitué par des atomes de fluor ; les résines de silicone ; et leurs mélanges.

Le composé siliconé peut représenter de 0,5 à 90 %, de préférence de 5 à 60 %, et mieux de 10 à 50 % du poids total de la composition.

La composition selon l'invention peut contenir au moins un composé non aqueux additionnel différent de l'huile hydrocarbonée non volatile, du composé siliconé non volatil et de l'agent dispersant hydrocarboné, choisi parmi les huiles, les corps gras pâteux à température ambiante, les cires, les gommes, les résines, et leurs mélanges.

En particulier, elle contient, en outre, au moins une cire. Par "cire" au sens de la présente invention, on entend un composé gras lipophile, solide à température ambiante (25°C), à changement d'état solide/liquide réversible, ayant une température de fusion supérieure à 30°C, et mieux supérieure à 45°C, pouvant aller jusqu'à 200° C, une dureté supérieure à 0,5 MPa, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ou l'huile de ricin hydrogénée ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 30°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone ou encore le tétrastéarate de di-(triméthylol-1,1,1 propane) fabriqué ou commercialisé par la société Hétérène sous la dénomination HEST 2T- 4S ; et leurs mélanges.

Les gommes utilisables dans l'invention se présentent généralement sous forme solubilisée dans une huile et les résines peuvent être liquides ou solides à température ambiante.

La nature et la quantité des gommes, corps pâteux ou cires sont fonction des propriétés mécaniques et des

textures recherchées. A titre indicatif, la composition peut contenir de 0,01 à 50 % en poids de cires, par rapport au poids total de la composition, de préférence de 2 à 40 %, et mieux de 5 à 30 %.

A titre d'exemple d'huiles additionnelles utilisables dans l'invention, on peut citer :

- les huiles hydrocarbonées d'origine animale telles que le perhydrosqualène ;
- les huiles hydrocarbonées d'origine végétale telles que les triglycérides liquides d'acides gras ayant de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stéarineries Dubois ou ceux vendus sous les dénominations Miglyol 810, 812 et 818 par la société Dynamit Nobel, l'huile de jojoba, de beurre de karité, le squalane d'origine synthétique ou végétale;
- les hydrocarbures linéaires ou ramifiés, d'origine minérale ou synthétique tels que les huiles de paraffine et leurs dérivés, la vaseline, les polydécènes, le polyisobutène hydrogéné tel que le Parléam®;
- les esters et les éthers de synthèse notamment d'acides gras comme les huiles de formule $R_1COOR_2$ dans laquelle $R_1$ représente le reste d'un acide gras supérieur comportant de 1 à 40 atomes de carbone et $R_2$ représente une chaîne hydrocarbonée contenant de 1 à 40 atomes de carbone avec $R_1 + R_2 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrityle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées comme le méthoxynonafluorobutane ;
- leurs mélanges.

Les huiles additionnelles de la composition peuvent représenter de 0,1 % à 90 % du poids total de la composition, de préférence de 5 à 60 % et mieux de 10 à 50 %.

La composition de l'invention peut comprendre, en outre, au moins un additif usuellement utilisé dans le domaine concerné, tel que des antioxydants, des conservateurs, des neutralisants, des gélifiants lipophiles, des dispersants, des actifs cosmétiques, et leurs mélanges. Ces additifs peuvent être présents dans la composition à raison de 0,0005 à 20% du poids total de la composition et mieux de 0,001 à 10%.

Par « actif cosmétique », on entend un composé lipophile ou hydrophile apportant un bénéfice aux matières kératiniques et plus spécialement à la peau et aux lèvres.

Comme actif cosmétique utilisable dans l'invention, on peut citer les vitamines A, E, C, $B_3$, F, les provitamines comme le D-panthénol, les actifs apaisants comme l'$\alpha$-bisabolol, l'aloe vera, l'allantoïne, les extraits de plantes ou les huiles essentielles, ies agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme compacté par exemple sous forme de coupelle utilisable par contact direct ou à l'éponge ou encore dans une bouillotte. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide. Elles peuvent alors constituer des fonds de teint ou des rouges à lèvres, des brillants à lèvres (gloss en terminologie anglo-saxonne), des produits solaires ou de coloration de la peau.

Les compositions de l'invention peuvent se présenter notamment sous forme de gel huileux, de liquide huileux, de pâte ou de stick ou encore sous forme de dispersion vésiculaire contenant des lipides ioniques et/ou non ioniques. Ces formes galéniques sont préparées selon les méthodes usuelles des domaines considérés.

La composition selon l'invention peut se présenter sous la forme d'une composition, colorée ou non de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant

les lèvres du froid et/ou du soleil et/ou du vent).

La composition de l'invention peut également se présenter sous la forme d'un produit de maquillage coloré de la peau, en particulier du visage comme un fond de teint, un blush, un fard à joues ou à paupières, de maquillage du corps comme un produit de tatouage semi-permanent, ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner.

De préférence, la composition selon l'invention se présente sous forme de rouge à lèvres ou de brillant à lèvres.

Bien entendu la composition de l'invention doit être physiologiquement acceptable (cosmétiquement 'acceptable), à savoir non toxique et susceptible d'être appliquée sur la peau, les phanères ou les lèvres d'êtres humains. Par « cosmétiquement acceptable », on entend agréable de goût, de toucher, d'aspect et/ou d'odeur.

L'invention est illustrée plus en détail dans les exemples suivants. Les pourcentages sont des pourcentages massiques.

**Exemples 1 et 2 : Sticks de rouges à lèvres**

Les compositions figurant dans le tableau (1) ci-après ont été réalisées.

La composition de l'exemple 1 selon l'invention comprend, comme agent dispersant selon l'invention, le monooléate de sorbitan ($\delta d$ = 17,32 $(J/cm^3)^{1/2}$ et $\delta a$ = 13,66 $(J/cm^3)^{1/2}$), fabriqué ou commercialisé par la société Uniquema sous la référence Span 80 V.

Dans la composition de l'exemple 2 (comparatif), les 13,4% de monooléate de sorbitan ont été substitués par 10% de triisostéarate de polyglycérol-2 fabriqué ou commercialisé par la société Nisshin Oil Mills sous la référence Salacos 43 ($\delta d$ = 16,7 $(J/cm^3)^{1/2}$ et $\delta a$ = 6,69 $(J/cm^3)^{1/2}$).

**Tableau (I)**

| Phase | | Exemple 1 (invention) | Exemple 2 (comparatif) |
|---|---|---|---|
| A | Triméllitate de tridécyle | 4,4 | 4,4 |
| | Copolymère vinylpyrrolidone/1-hexadécène commercialisé ou fabriqué par la société ISP sous la référence Antaron V-216 | 1,6 | 1,6 |
| | Copolymère vinylpyrrolidone/1-eicosène commercialisé ou fabriqué par la société ISP sous la référence Antaron V-220 | 0,8 | 0,8 |
| | Bis diglycéryl polyacyladipate-2 | 1,6 | 1,6 |
| | BHT | 0,04 | 0,04 |
| | Triisostéarate de polyglycérol-2 | - | 13,4 |
| | Monooléate de sorbitan | 13,4 | - |
| | Isononanoate d'isononyle | 10 | 10 |
| B- | Cire de polyéthylène (MM = 500 g/mol). | 6,6 | 6,6 |
| | Stéarate d'octacosanyle | 5,5 | 5,5 |
| C- | Red 21 | 0,06 | 0,06 |
| | Red 7 | 0,2 | 0,2 |
| | Iron oxydes (CI 77491 et CI 77499) | 2,2 | 2,2 |
| C'- | Copolymère di méthacrylate d'éthylène glycol/méthacrylate de lauryle fabriqué ou commercialisé sous la référence Polytrap 603 par la société Advanced Polymer Systems | 1 | 1 |
| | N-lauroyl L-lysine | 2,5 | 2,5 |
| | Kaolin | 5 | 5 |

(suite)

| Phase | | Exemple 1 (invention) | Exemple 2 (comparatif) |
|---|---|---|---|
| **D-** | Isoparaffine hydrogénée fabriqué ou commercialisé sous la référence Parléam par la société Nippon Oil and Fats | qsp 100 | qsp 100 |
| | Polyisobutène hydrogéné fabriqué ou commercialisé sous la référence Panalane H-300E par la société Amoco Chemical | 8,5 | 8,5 |
| | Phényltrimethicone commercialisé ou fabriqué par la société Dow Corning sous la référence DC 556 | 12,7 | 12,7 |
| | Silice pyrogénée | 3 | 3 |
| **E-** | Mica oxyde de titane | 1,8 | 1,8 |

Mode opératoire :

Les pigments (phase C) et les charges (phase C') sont broyés dans la phase A.
Parallèlement, un gel de silice est préparé (phase D) en mélangeant la silice dans l'isoparaffine hydrogénée, la phényltriméthicone et le polyisobutène hydrogéné.
Puis le broyat (phases A + C + C') ainsi que le gel de silice (phase D) et les cires (phase B), sont ajoutés dans un poêlon et chauffés à 100°C pendant 2 heures, et homogénéisé.
Enfin, la nacre (phase E) est ajoutée au mélange que l'on coule dans un moule approprié à 42°C. Le moule est ensuite placé à - 20°C pendant une demi-heure, puis on procède au démoulage des sticks.

Evaluation cosmétique :

La tenue des deux formules a été évaluée à l'aide de méthodes instrumentales et sensorielles sur un panel de 12 personnes qualifiées qui ont appliqué chacune des formules l'une après l'autre.
L'évaluation de la tenue se déroule de la façon suivante :

- dans un premier temps, une évaluation de la tenue est réalisée une heure après l'application de la formule sur les lèvres.
- dans un second temps, la tenue est évaluée après une série d'épreuves consistant à faire deux « bises » sur un mouchoir en papier, boire une boisson chaude puis une boisson froide et manger 4 bouchées d'un sandwich et une pomme.

La tenue instrumentale est évaluée sur une échelle allant de 1 à 100 : 1 correspond à une formule qui ne tient pas du tout et 100 à une formule qui tient très bien. La différence entre deux résultats est significative si elle est supérieure ou égale à 10.
La migration, la brillance et le confort ont aussi été évalués par les 12 personnes :

- la brillance a été évaluée juste après l'application de la formule puis au bout d'une heure
- la migration et le confort ont été évalués au bout d'une heure.
  La composition de l'exemple 1 selon l'invention possède de meilleures propriétés de tenue que la composition de l'exemple 2 (la tenue a été évaluée à 71 pour la composition de l'exemple 1 selon l'invention contre une valeur de 59 pour la composition de l'exemple 2), tout en étant de brillance, de confort et de migration équivalentes. De plus, la brillance du film de la composition de l'exemple 1 persiste plus longtemps.

**Exemples 3 et 4 : Sticks de rouges à lèvres**

Les inventeurs ont comparé les propriétés de deux compositions selon l'invention et l'art antérieur. La composition de l'exemple 3 selon l'invention contient un agent dispersant hydrocarboné qui est le monoisostéarate de polyglycérol-2 (fabriqué ou commercialisé par la société Nisshin Oils Mills sous la référence Salacos 41), une huile hydrocarbonée qui est l'isononyl isononanoate de masse molaire égale à 284,4 g/mol (fabriquée ou commercialisée par la société Stéarineries Dubois) et une phase particulaire inerte comprenant du kaolin et de la lauroyl lysine. La composition

de l'exemple 4 (comparatif) contient également comme agent dispersant hydrocarboné le monoisostéarate de polyglycérol-2 mais ne contient pas d'isononyl isononanoate ni de kaolin et de lauroyl lysine.

**Tableau (II)**

| **Phase** | | **Exemple 3 (invention)** | **Exemple 4 (comparatif)** |
|---|---|---|---|
| **A -** | Triméllitate de tridécyle | 4,4 | 9,5 |
| | Copolymère vinylpyrrolidone/1-hexadécène commercialisé ou fabriqué par la société ISP sous la référence Antaron V-216 | 1,6 | 4 |
| | Copolymère vinylpyrrolidone/1-eicosène commercialisé ou fabriqué par la société ISP sous la référence Antaron V-220 | 0,8 | 2 |
| | Bis diglycéryl polyacyladipate-2. | 1,6 | 4 |
| | BHT | 0,02 | 0,05 |
| | Monoisostéarate de polyglycérol-2 | 10 | 10 |
| | Triisostéarate de polyglycérol-2 | 3,4 | 0,7 |
| | Isononyl isononanoate | 10 | |
| **B-** | Cire de polyéthylène (MM= 500 g/mol) | 6 | 6 |
| | Stéarate d'octa-cosanyle | 5 | 5 |
| **C-** | Red 21 | 0,06 | 0,06 |
| | Red 7 | 0,2 | 0,2 |
| | Iron oxydes | 2,2 | 2,2 |
| **C'-** | Copolymère di méthacrylate d'éthylène glycol/méthacrylate de lauryle fabriqué ou commercialisé sous la référence Polytrap 603 par la société Advanced Polymer Systems | 1 | 1 |
| | N-lauroyl L-lysine | 2,5 | - |
| | Kaolin | 5 | - |
| **D-** | Isoparaffine hydrogénée fabriqué ou commercialisé sous la référence Parléam par la société Nippon Oil and Fats | qsp 100 | qsp 100 |
| | Polybutylène | | 10,7 |
| | Polyisobutène hydrogéné fabriqué ou commercialisé sous la référence Panalane H-300E par la société Amoco Chemical | 8,7 | - |
| | Phényltrimethicone commercialisé ou fabriqué par la société Dow Corning sous la référence DC 556 | 13,1 | 16 |
| | Silice pyrogénée | 3,1 | 3,1 |
| **E-** | Mica oxyde de titane | 1,8 | 1,8 |

Le mode opératoire est le même que celui des exemples 1 et 2 ci-dessus.

<u>Evaluation cosmétique</u>

- La tenue, la migration, la brillance et le confort ont été évalués selon les mêmes méthodes que celles des exemples

1 et 2.

La composition de l'exemple 3 selon l'invention a été jugée brillante, confortable et possède des propriétés de tenue et de non migration supérieures à celles de la composition de l'exemple 4.

**Exemples 5 et 6 : Sticks de rouges à lèvres**

Les inventeurs ont comparé les propriétés de deux compositions selon l'invention et l'art antérieur. La composition de l'exemple 5 selon l'invention contient un agent dispersant hydrocarboné qui est le monoisostéarate de polyglycérol-2 (fabriqué ou commercialisé par la société Nisshin Oils Mills sous la référence Salacos 41) et la composition de l'exemple 6 (comparatif) contient un agent dispersant siliconé qui est le cétyl diméthicone copolyol (fabriqué ou commercialisé par la société Goldschmidt sous la référence Abil EM 90 Desodorise).

**Tableau (III)**

| Phase | | Exemple 5 (invention) | Exemple 6 (comparatif) |
|---|---|---|---|
| A - | Triméllitate de tridécyle | 4,4 | 4,4 |
| | Copolymère vinylpyrrolidone/1-hexadecene commercialisé ou fabriqué par la société ISP sous la référence Antaron V-216 | 1,6 | 1,6 |
| | Copolymère vinylpyrrolidone/1-eicosène commercialisé ou fabriqué par la société ISP sous la référence Antaron V-220 | 0,8 | 0,8 |
| | Bis diglycéryl polyacyladipate-2. | 1,6 | 1,6 |
| | BHT | 0,04 | 0,04 |
| | Triisostéarate de polyglycéryle-2 | 3,4 | 3,4 |
| | Cétyl diméthicone copolyol | - | 10 |
| | Monoisostéarate de polyglycerol-2 | 10 | - |
| | Isononanoate d'isononyle | 10 | 10 |
| B- | Cire de polyéthylène (MM= 500 g/mol) | 6,6 | 6,6 |
| | Stéarate d'octa-cosanyle | 5,5 | 5,5 |
| C- | Red 21 | 0,06 | 0,06 |
| | Red 7 | 0,2 | 0,2 |
| | Iron oxydes | 2,2 | 2,2 |
| C'- | Copolymère di méthacrylate d'éthylène glycol/méthacrylate de lauryle fabriqué ou commercialisé sous la référence Polytrap 603 par la société Advanced Polymer Systems | 1 | 1 |
| | N-lauroyl L-lysine | 2,5 | 2,5 |
| | Kaolin | 5 | 5 |
| D- | Isoparaffine hydrogénée fabriqué ou commercialisé sous la référence Parléam par la société Nippon Oil and Fats | qsp 100 | qsp 100 |
| | Polyisobutène hydrogéné fabriqué ou commercialisé sous la référence Panalane H-300E par la société Amoco Chemical | 8,5 | 8,5 |
| | Phényltrimethicone commercialisé ou fabriqué par la société Dow Corning sous la référence DC 556 | 12,7 | 12,7 |
| | Silice pyrogénée | 3 | 3 |

(suite)

| Phase | | Exemple 5 (invention) | Exemple 6 (comparatif) |
|---|---|---|---|
| E- | Mica oxyde de titane | 1,8 | 1,8 |

Le mode opératoire est le même que celui des exemples 1 et 2 ci-dessus.

Evaluation cosmétique

- La tenue, la migration, la brillance et le confort ont été évalués selon les mêmes méthodes que celles des exemples 1 et 2.

La composition de l'exemple 5 selon l'invention a été jugée brillante, confortable, peu migrante et possède une tenue nettement supérieure à celle de la composition de l'exemple 6 (la tenue après épreuve a été évaluée à 71 pour la composition 5 selon l'invention contre 49 pour la composition de l'exemple 6).

## Revendications

**1.** Composition anhydre de soin ou de maquillage des matières kératiniques, comprenant un milieu cosmétiquement acceptable contenant

- au moins une huile hydrocarbonée non volatile, ladite huile non volatile étant un ester ramifié et saturé,
- au moins une phase particulaire contenant au moins une charge inerte et
- au moins un agent dispersant hydrocarboné comportant des atomes de carbone et d'hydrogène et une ou plusieurs fonctions choisies parmi les fonctions hydroxyle, ester, éther, carboxylique, amide, ledit agent dispersant présentant les paramètres de solubilité δd et δa satisfaisant aux conditions suivantes : $16,2 \leq \delta_d \leq 20$ $(J/cm^3)^{1/2}$ et $9,1 \leq \delta_a \leq 20$ $(J/cm^3)^{1/2}$, ledit agent dispersant hydrocarboné représentant de 0,5 à 40% du poids total de la composition,

ladite composition comprenant moins de 10% d'huile volatile par rapport au poids total de la composition.

**2.** Composition selon la revendication 1, **caractérisée en ce qu**'elle contient moins de 5% d'huile volatile par rapport au poids total de la composition, et mieux moins de 2%.

**3.** Composition selon la revendication 1, **caractérisée en ce que** l'agent dispersant hydrocarboné présente les paramètres de solubilité δd et δa satisfaisant aux conditions suivantes : $16, 3 \leq \delta_d \leq 19$ $(J/cm^3)^{1/2}$ et $10 \leq \delta_a \leq 18,1$ $(J/cm^3)^{1/2}$.

**4.** Composition selon l'une des revendications précédentes, **caractérisée en ce que** l'agent dispersant hydrocarboné présente les paramètres de solubilité δd et δa satisfaisant aux conditions suivantes : $16,9 \leq \delta_d \leq 18$ $(J/cm^3)^{1/2}$ et $13 \leq \delta_a \leq 14,5$ $(J/cm^3)^{1/2}$.

**5.** Composition selon l'une des revendications précédentes, **caractérisée en ce que** l'agent dispersant hydrocarboné est fluide à température ambiante (25°C) et/ou présente un indice de réfraction ≥ 1,45 à 20°C.

**6.** Composition selon l'une des revendications précédentes, **caractérisée en ce que** l'agent dispersant hydrocarboné présente une structure chimique comportant au moins un groupement polaire choisi parmi -COOH : -OH ; oxyde d'éthylène : $-(O-CH_2-CH_2-)$; oxyde de propylène

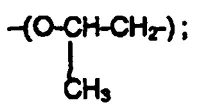

$-PO_4$ ; NHR; $NR_1R_2$ avec $R_1$, $R_2$ formant éventuellement un cycle et représentant un radical alkyle ou alkoxy

linéaire ou ramifié en $C_1$ à $C_{20}$ ou

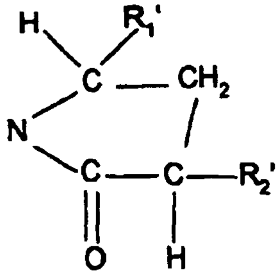

avec $R_1$' et $R_2$' qui peuvent être égal à H ou à une chaîne alkyle ou alkoxy linéaire ou ramifiée en $C_1$ à $C_{20}$.

7. Composition selon l'une des revendications précédentes, **caractérisée en ce que** le dispersant hydrocarboné est choisi parmi :

- les alcools gras modifiés éther et en particulier les produits d'addition de l'oxyde d'éthylène et / ou de l'oxyde de propylène avec i) un alcool gras linéaire ou ramifié ou avec ii) un alkylphénol,
- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le potyglycérol, le pentaérythritol, les alcools saccharidiques tels que le sorbitol, et en particulier les esters de polyglycérol,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un adde gras saturé ou insaturé avec au moins un produit d'addition de l'oxyde d'étttylène et du sorbitan,
- les produits d'addition de l'oxyde d'éthylène avec l'huile de ricin et / ou l'huile de ricin hydrogénée ,
- les phosphate trialkylés et les mono, dl et tripliosphate aikylés,

et leurs mélanges.

8. Composition selon l'une des revendications précédentes, **caractérisée en ce que** le dispersant hydrocarboné est choisi parmi les monoesters, les diesters et les esters résultant d'une estérification partielle.

9. Composition selon l'une des revendications précédentes, **caractérisée en ce que** le dispersant hydrocarboné est choisi parmi :

- les produits d'addition de 2 à 30 moles d'oxyde d'éthylène et / ou de 0 à 5 moles d'oxyde de propylène avec 1) un alcool gras linéaire ou ramifié en $C_8$ à $C_{40}$, et mieux en $C_8$ à $C_{22}$

ou avec ll) un alkylphénol,

- les esters résultant de la réaction d'au moins un acide gras en $C_8$ à $C_{40}$, et mieux en $C_8$ à $C_{22}$, avec au moins un produit d'addition de 1 à 30 moles d'oxyde d'éthylène et du glycérol ou avec au moins un produit d'addition de 1 à 30 moles d'oxyde d'éthylène et du polyglycérol,
- les esters résultant de la réaction du glycérol ou du polyglycérol avec au moins un produit d'addition de 2 à 30 moles d'oxyde d'éthylène et d'un adde gras saturé ou insaturé en $C_8$ à $C_{40}$, et mieux en $C_8$ à $C_{22}$,
- les esters partiels résultant de la réaction d'au moins un acide gras linéaire ou ramifié, saturé ou insaturé en $C_8$ à $C_{40}$ et mieux en $C_8$ à $C_{22}$, d'acide ricinoléique ou d'acide 12-hydroxystéarique, avec le glycérol, le polyglycérol, le pentaérythritol ou le sorbitol,
- les esters résultant de la réaction du sorbitan avec au moins un acide gras linéaire ou ramifié, saturé ou insaturé en $C_8$ à $C_{40}$ et mieux en $C_8$ à $C_{22}$,

- les esters résultant iii) de la réaction du sorbitan avec au moins un produit d'addition de 2 à 30 moles d'oxyde d'éthylène et d'un acide gras saturé ou insaturé en $C_8$ à $C_{40}$ et mieux en $C_8$ à $C_{22}$ ou iv) de la réaction d'au moins un acide gras saturé ou insaturé en $C_8$ à $C_{40}$ et mieux en $C_8$ à $C_{22}$, avec au moins un produit d'addition-de 2 à 30 moles d'oxyde d'éthylène et du sorbitan,
- les produits d'addition de 2 à 60 moles d'oxyde d'éthylène avec l'huile de ricin et 1 ou l'huile de ricin hydrogénée,
- les phosphate trialkylés et les mono, di et triphosphate alkylés,
- et leurs mélanges.

**10.** Composition selon l'une des revendications précédentes, **caractérisée en ce que** le dispersant hydrocarboné est choisi parmi : l'alcool de myristyle oxyéthyténé à 15 OE, le monoisostéarate de polyglycérol-2 oxyéthyléné à 5 OE, le diisostéarate de polyglycérol-3, le monoisostéarate de glycérol, le monoisostéarate de polyglycérol-2, l'isostéarate de polyglycérol-3, l'isostéarate de polyglycérol-4, le monolsostéarate de polyglycérol-6, le monoisostéarate de polyglycérol-10, le monooléate de polyglycérol-2, l'isostéarate de sorbitan, le monooléate de sorbitan, le monooléate de sorbitan oxyéthyléné à 5 OE, et leurs mélanges.

**11.** Composition selon l'une des revendications précédentes, **caractérisée en ce que** le dispersant hydrocarboné est choisi parmi les esters partiels de polyglycérol et d'acide isostéarique, les esters partiels de polyglycérol et d'acide oléique, les esters partiels de sorbitan et d'acide oléique, et leurs mélanges.

**12.** Composition selon l'une des revendications précédentes, **caractérisée en ce que** le dispersant hydrocarboné est choisi parmi le diisostéarate de polyglycérol-3, le monolsostéarate de polyglycérol-2, le monooléate de polyglycérol-2, le monooléate de sorbitan, et leurs mélanges.

**13.** Composition selon l'une des revendications précédentes, **caractérisée en ce que** l'agent dispersant hydrocarboné représente de 3 à 20% et mieux de 5 à 15% du poids total de la composition.

**14.** Composition selon l'une des revendications précédentes, **caractérisée en ce que** l'huile hydrocarbonée non volatile a une masse molaire allant de 200 à 1500 g/mol.

**15.** Composition selon l'une des revendications précédentes, **caractérisée en ce que** l'huile hydrocarbonée non volatile a une masse molaire allant de 220 à 500 g/mol.

**16.** Composition selon l'une des revendications précédentes, **caractérisée en ce que** l'huile hydrocarbonée non volatile a une masse molaire allant de 230 à 430 g/mol.

**17.** Composition selon l'une des revendications précédentes, **caractérisée en ce que** l'hulle hydrocarbonée non volatile est choisie parmi les esters résultant de la réaction d'un acide carboxylique ramifié, saturé en $C_2$ à $C_{30}$, et mieux en $C_2$ à $C_{18}$ avec un alcool en $C_2$ à $C_{30}$, et mieux en $C_2$ à $C_{20}$ ou avec un polyol en $C_2$ à $C_{20}$ et mieux en $C_2$ à $C_8$, et leurs mélanges.

**18.** Composition selon l'une des revendications précédentes, **caractérisée en ce que** l'huile hydrocarbonée non volatile est choisie parmi les esters de l'acide néopentanoïque, les esters de l'acide isononanoïque et leurs mélanges.

**19.** Composition selon l'une des revendications précédentes, **caractérisée en ce que** l'huile hydrocarbonée non volatile est choisie parmi l'isodecyl neopentanoate, l'isotridecyl neopentanoate, l'isostearyl neopentanoate, l'octyldodecyl neopentanoate, l'isononyl isononanoate, l'octyl isononanoate, l'isodecyl isononanoate, l'isotridecyl isononanoate, l'isostéaryl isononanoate et leurs mélanges.

**20.** Composition selon l'une des revendications précédentes, **caractérisée en ce que** l'huile hydrocarbonée non volatile représente de 5 à 98 %, de préférence de 7 à 60 %, mieux de 10 à 50 %, et encore mieux de 10 à 30% du poids total de la composition.

**21.** Composition selon l'une des revendications précédentes, **caractérisée en ce que** la phase particulaire Inerte contient au moins une charge inerte absorbante ou non.

**22.** Composition selon la revendication précédente, **caractérisée en ce que** la charge inerte est choisie parmi les charges minérales ou organiques, lamellaires, sphériques ou oblongues.

**23.** Composition selon la revendication 21 ou 22, **caractérisée en ce que** la charge inerte est choisie parmi le talc, le mica, la silice, le kaolin, les poudres de polyamide, de poly-β-alanine et de polyéthylène, les poudres de polytétrafluoroéthylène, la lauroyl-lysine, l'amidon, le nitrure de bore, les microsphères creuses polymériques, les copolymères d'acide acrylique et les microbilles de résine de silicone, le carbonate de calcium précipité, le dicalcium phosphate, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc. de magnésium ou de lithium, le laurate de zinc, le myristate de magnésium, et leurs mélanges.

**24.** Composition selon l'une des revendications précédentes, **caractérisée en ce que** la phase particulaire inerte représente de 0,1 à 30 % du poids total de la composition, mieux de 2 à 25 % et encore mieux 3 à 20 %.

**25.** Composition selon l'une des revendications précédentes, **caractérisée en ce qu**'elle contient au moins une matière colorante.

**26.** Composition selon la revendication précédente, **caractérisée en ce que** la matière colorante comprend au moins un composé pulvérulent choisi parmi les pigments, les nacres, et leurs mélanges.

**27.** Composition selon la revendication 25 ou 26, **caractérisée en ce que** la matière colorante représente de 0,001 à 50%, de préférence de 0,01 à 40 % et mieux de 0,05 à 30%, par rapport au poids total de la composition.

**28.** Composition selon l'une des revendications précédentes, **caractérisée en ce qu**'elle comprend un composé siliconé non volatil.

**29.** Composition selon la revendication précédente, **caractérisée en ce que** la composé siliconé non volatil est choisi parmi les composés liquides à température ambiante.

**30.** Composition selon la revendication 28 ou 29, **caractérisée en ce que** le composé siliconé non volatil présente une viscosité allant de 5 à 100 000 cSt à 25°C, de préférence allant de 10 à 50 000 cSt et mieux de 10 à 5 000 cSt.

**31.** Composition selon l'une des revendications 28 à 30, **caractérisée en ce que** le composé siliconé non volatil est choisi parmi les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les silicones fluorées comportant un groupement fluoré pendant ou en bout de chaîne siliconée ayant de 1 à 12 atomes de carbone dont tout ou partie des hydrogènes est substitué par des atomes de fluor ; les résines de silicone; et leurs mélanges.

**32.** Composition selon l'une des revendications 28 à 31, **caractérisée en ce que** le composé siliconé non volatil représente de 0,5 à 90 %, de préférence de 5 à 60 % et mieux de 10 à 50 % du poids total de la composition.

**33.** Composition selon l'une des revendications précédentes, **caractérisée en ce qu**'elle contient, en outre, au moins une cire.

**34.** Composition selon la revendication précédente, **caractérisée en ce que** la cire représente de 0,01 à 50 %, de préférence de 2 à 40 %, et mieux de 5 à 30 % du poids total de la composition.

**35.** Composition selon l'une des revendications précédentes, **caractérisée en ce qu'**elle contient, en outre, au moins un additif choisi parmi les antioxydants, les conservateurs, les neutralisants, les gélifiants lipophiles ou de composés non aqueux liquides, les dispersants, les actifs cosmétiques, et leurs mélanges.

**36.** Composition selon l'une des revendications précédentes, **caractérisée en ce qu**'elle contient au moins un actif cosmétique choisi parmi les vitamines (A, E, C, $B_3$, F) les provitamines, les actifs apaisants, les extraits de plantes ou les huiles essentielles, les agents protecteurs ou restructurants, les actifs fraîcheur, les émollients, les hydratants, les actifs antirides, les acides gras essentiels, et leurs mélanges.

**37.** Composition selon l'une des revendications précédentes, **caractérisée en ce qu**'elle se présente sous forme d'un produit coulé ou compacté.

**38.** Composition de maquillage en particulier de la peau ou des lèvres, **caractérisée en ce qu**'elle est conforme à l'une des revendications précédentes.

**39.** Composition selon l'une des revendications précédentes, **caractérisée en ce qu**'elle se présente sous forme d'un rouge à lèvres ou d'un brillant à lèvres.

**40.** Procédé cosmétique pour conférer à un film de composition cosmétique anhydre des propriétés de tenue, de brillant, de confort et/ou de non-migration, consistant à introduire dans ladite composition au moins huile hydrocarbonée non volatile, ladite huile non volatile étant un ester ramifié et saturé, au moins une phase particulaire inerte et au moins un agent dispersant hydrocarboné qui présente les paramètres de solubilité δd et δa satisfaisant aux conditions suivantes : $16{,}2 \leq \delta_d \leq 20$ $(J/cm^3)^{1/2}$ et $9{,}1 \leq \delta_a \leq 20$ $(J/cm^3)^{1/2}$, ledit agent dispersant comportant des atomes de carbone et d'hydrogène et une ou plusieurs fonctions choisies parmi les fonctions hydroxyle, ester, éther, carboxylique, amide, ledit agent dispersant hydrocarboné représentant de 0,5 à 40% du poids total de la composition,
ladite composition comprenant moins de 10% d'huile volatile par rapport au poids total de la composition.

**41.** Procédé selon la revendication précédente, **caractérisé en ce que** l'agent dispersant hydrocarboné est choisi parmi :

- les alcools gras modifiés éther et en particulier les produits d'addition de l'oxyde d'éthylène et / ou de l'oxyde de propylène avec I) un alcool gras linéaire ou ramifié ou avec ii) un alkylphénol,
- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'adde ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol ,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et / ou l'huile de ricin hydrogénée,
- les phosphate trialkylés et les mono, di et triphosphate alkylés,

et leurs mélanges.

**42.** Procédé selon la revendication 40 ou 41, **caractérisé en ce que** l'huile hydrocarbonée non volatile a une masse molaire allant de 200 à 1500 g/mol.

**43.** Procédé selon l'une des revendications 40 à 42, **caractérisé en ce que** l'huile hydrocarbonée non volatile est choisie parmi les esters résultant de la réaction d'un acide carboxylique ramifié, saturé en $C_2$ à $C_{30}$, et mieux en $C_2$ à $C_{18}$ avec un alcool en $C_2$ à $C_{30}$, et mieux en $C_2$ à $C_{20}$ ou avec un polyol en $C_2$ à $C_{20}$ et mieux en $C_2$ à $C_8$, et leurs mélanges.

**44.** Procédé selon l'une des revendications 40 à 42, **caractérisé en ce que** l'huile hydrocarbonée non volatile est choisie parmi les esters de l'acide néopentanoïque, les esters de l'acide isononanoïque et leurs mélanges.

**45.** Procédé selon l'une des revendications 40 à 43, **caractérisé en ce que** la phase particulaire inerte est choisie parmi le talc, le mica, la silice, le kaolin, les poudres de polyamide, de poly-β-alanine et de polyéthylène, les poudres de polytétrafluoroéthylène, la lauroyl-lysine, l'amidon, le nitrure de bore, les microsphères creuses polymériques, les copolymères d'acide acrylique et les microbilles de résine de silicone, le carbonate de calcium précipité, le dicalcium phosphate, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

**46.** Utilisation de l'association d'au moins une huile hydrocarbonée non volatile, 1-<> d'au moins une phase particulaire inerte et d'au moins un agent dispersant hydrocarboné comportant des atomes de carbone et d'hydrogène et une

ou plusieurs fonctions choisies parmi les fonctions hydroxyle, ester, éther, carboxylique, amide, ledit agent dispersant présentant les paramètres de solubilité δd et δa satisfaisant aux conditions suivantes : $16,2 \leq \delta d \leq 20\ (J/cm^3)^{1/2}$ et $9,1 \leq \delta_a \leq 20\ (J/cm^3)^{1/2}$, dans une composition anhydre de bonne tenue, brillante, confortable et lou non migrante, 2-<>, 3-<>

**47.** Utilisation de l'association d'au moins une huile hydrocarbonée non volatile, ladite huile non volatile étant un ester ramifié et saturé, d'au moins une phase particulaire inerte et d'au moins un agent dispersant hydrocarboné comportant des atomes de carbone et d'hydrogène et une ou plusieurs fonctions choisies parmi les fonctions hydroxyle, ester, éther, carboxylique, amide, ledit agent dispersant présentant les paramètres de solubilité δd et δa satisfaisant aux conditions suivantes : $16,2 \leq \delta_d \leq 20\ (J/cm^3)^{1/2}$ et $9,1 \leq \delta_a \leq 20\ (J/cm^3)^{1/2}$ dans une composition cosmétique anhydre, comme agent pour conférer à ladite composition des propriétés de tenue, de brillant, de confort et/ou de non-migration, ledit agent dispersant hydrocarboné représentant de 0,5 à 40% du poids total de la composition,
ladite composition comprenant moins de 10% d'huile volatile par rapport au poids total de la composition.

**48.** Utilisation selon la revendication 46 ou 47, **caractérisée en ce que** l'agent dispersant hydrocarboné est choisi parmi :

- les alcools gras modifiés éther et en particulier les produits d'addition de l'oxyde d'éthylène et / ou de l'oxyde de propylène avec i) un alcool gras linéaire ou ramifié ou avec ii) un alkylphénol,
- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou Insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et / ou l'huile de ricin hydrogénée,
- les phosphate trialkylés et les mono, di et triphosphate alkylés,

et leurs mélanges.

**49.** Utilisation selon l'une des revendications 46 à 48, **caractérisé en ce que** l'huile hydrocarbonée non volatile a une masse molaire allant de 200 à 1500 g/mol.

**50.** Utilisation selon l'une des revendications 46 à 49, **caractérisée en ce que** l'huile hydrocarbonée non volatile est choisie parmi les esters résultant de la réaction d'un acide carboxylique ramifié, saturé en $C_2$ à $C_{30}$, et mieux en $C_2$ à $C_{18}$ avec un alcool en $C_2$ à $C_{30}$, et mieux en $C_2$ à $C_{20}$ ou un avec polyol en $C_2$ à $C_{20}$ et mieux en $C_2$ à $C_8$, et leurs mélanges.

**51.** Utilisation selon l'une des revendications 46 à 50, **caractérisée en ce que** l'huile hydrocarbonée non volatile est choisie parmi les esters de l'acide néopentanoïque, les esters de l'acide isononanoïque et leurs mélanges.

**52.** Utilisation selon l'une des revendications 46 à 51, **caractérisée en ce que** la phase particulaire inerte est choisie parmi le talc, le mica, la silice, le kaolin, les poudres de polyamide, de poly-β-alanine et de polyéthylène, les poudres de polytétrafluoroéthylène, la lauroyl-lysine, l'amidon, le nitrure de bore, les microsphères creuses polymériques, les copolymères d'acide acrylique et les microbilles de résine de silicone, le carbonate de calcium précipité, le dicalcium phosphate, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

## Claims

1. Anhydrous care or makeup composition for keratin materials which comprises a cosmetically acceptable medium comprising

   - at least one non-volatile hydrocarbon oil, the said non-volatile oil being a branched and saturated ester,
   - at least one particulate phase comprising at least one inert filler and
   - at least one hydrocarbon dispersant containing carbon and hydrogen atoms and one or more functional groups selected from hydroxyl, ester, ether, carboxylic and amide fucntions, the said dispersant having solubility parameters δd and δa which meet the following conditions: $16.2 \leq \delta_d \leq 20\ (J/cm^3)^{1/2}$ and $9.1 \leq \delta_a \leq 20\ (J/cm^3)^{1/2}$, the said hydrocarbon dispersant representing from 0.5 to 40% of the total weight of the composition,

   the said composition comprising less than 10% of volatile oil relative to the total weight of the composition.

2. Composition according to Claim 1, **characterized in that** it contains less than 5% of volatile oil relative to the total weight of the composition, and more preferably less than 2%.

3. Composition according to Claim 1, **characterized in that** the hydrocarbon dispersant has solubility parameters δd and δa which meet the following conditions: $16.\ 3 \leq \delta_d \leq 19\ (J/cm^3)^{1/2}$ and $10 \leq \delta_a \leq 18.1\ (J/cm^3)^{1/2}$.

4. Composition according to one of the preceding claims, **characterized in that** the hydrocarbon dispersant has solubility parameters $\delta_d$ and $\delta_a$ which meet the following conditions: $16.9 \leq \delta_d \leq 18\ (J/cm^3)^{1/2}$ and $13 \leq \delta_a \leq 14.\ 5\ (J/cm^3)^{1/2}$.

5. Composition according to one of the preceding claims, **characterized in that** the hydrocarbon dispersant is fluid at room temperature (25°C) and/or has a refractive index ≥ 1.45 at 20°C.

6. Composition according to one of the preceding claims, **characterized in that** the hydrocarbon dispersant has a chemical structure which includes at least one polar group selected from -COOH; -OH; ethylene oxide; $-(O-CH_2-CH_2-)$; propylene oxide

   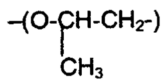 ;

   $-PO_4$; NHR; $NR_1R_2$ where $R_1$ and $R_2$ optionally form a ring and represent a linear or branched $C_1$ to $C_{20}$ alkyl or alkoxy radical, or

   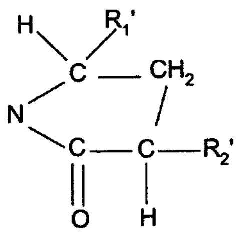

   where $R_1$' and $R_2$' can be H or a linear or branched $C_1$ to $C_{20}$ alkyl or alkoxy chain.

7. Composition according to one of the preceding claims, **characterized in that** the hydrocarbon dispersant is selected from:

   - ether-modified fatty alcohols and in particular the addition products of ethylene oxide and/or propylene oxide with i) a linear or branched fatty alcohol or with ii) an alkylphenol,
   - esters resulting from the reaction of at least one fatty acid with at least one addition product of ethylene oxide and glycerol or with at least one addition product of ethylene oxide and polyglycerol,
   - esters resulting from the reaction of glycerol or polyglycerol with at least one addition product of ethylene oxide and a saturated or unsaturated fatty acid,

- partial esters resulting from the reaction of at least one saturated or unsaturated, linear or branched fatty acid, ricinoleic acid or 12-hydroxystearic acid with at least one polyol such as glycerol, polyglycerol, pentaerythritol, saccharide alcohols such as sorbitol, and especially esters of polyglycerol,
- esters resulting from the reaction of sorbitan with at least one saturated or unsaturated, linear or branched fatty acid,
- ether-modified sorbitan esters, and especially esters resulting iii) from the reaction of sorbitan with at least one addition product of ethylene oxide and a saturated or unsaturated fatty acid or iv) from the reaction of at least one saturated or unsaturated fatty acid with at least one addition product of ethylene oxide and sorbitan,
- addition products of ethylene oxide with castor oil and/or hydrogenated castor oil,
- trialkyl phosphates and alkyl mono-, di- and triphosphates,

and mixtures thereof.

**8.** Composition according to one of the preceding claims, **characterized in that** the hydrocarbon dispersant is selected from monoesters, diesters and esters resulting from a partial esterification.

**9.** Composition according to one of the preceding claims, **characterized in that** the hydrocarbon dispersant is selected from:

- addition products of from 2 to 30 moles of ethylene oxide and/or from 0 to 5 moles of propylene oxide with i) a linear or branched $C_8$ to $C_{40}$, more preferably $C_8$ to $C_{22}$, fatty alcohol or with ii) an alkylphenol,
- esters resulting from the reaction of at least one $C_8$ to $C_{40}$, more preferably $C_8$ to $C_{22}$, fatty acid with at least one addition product of from 1 to 30 moles of ethylene oxide and glycerol or with at least one addition product of from 1 to 30 moles of ethylene oxide and polyglycerol,
- esters resulting from the reaction of glycerol or polyglycerol with at least one addition product of from 2 to 30 moles of ethylene oxide and a $C_8$ to $C_{40}$, more preferably $C_8$ to $C_{22}$, saturated or unsaturated fatty acid,
- partial esters resulting from the reaction of at least one saturated or unsaturated $C_8$ to $C_{40}$, more preferably $C_8$ to $C_{22}$, linear or branched fatty acid, ricinoleic acid or 12-hydroxystearic acid with glycerol, polyglycerol, pentaerythritol or sorbitol,
- esters resulting from the reaction of sorbitan with at least one saturated or unsaturated $C_8$ to $C_{40}$, more preferably $C_8$ to $C_{22}$, linear or branched fatty acid,
- esters resulting iii) from the reaction of sorbitan with at least one addition product of from 2 to 30 moles of ethylene oxide and a saturated or unsaturated $C_8$ to $C_{40}$, more preferably $C_8$ to $C_{22}$, fatty acid or iv) from the reaction of at least one $C_8$ to $C_{40}$, more preferably $C_8$ to $C_{22}$, saturated or unsaturated fatty acid with at least one addition product of from 2 to 30 moles of ethylene oxide and sorbitan,
- addition products of from 2 to 60 moles of ethylene oxide with castor oil and/or hydrogenated castor oil,
- trialkyl phosphates and alkyl mono-, di- and triphosphates,
- and mixtures thereof.

**10.** Composition according to one of the preceding claims, **characterized in that** the hydrocarbon dispersant is selected from: ethoxylated myristyl alcohol containing 15 EO, ethoxylated polyglyceryl-2 monoisostearate containing 5 EO, polyglyceryl-3 diisostearate, glyceryl monoisostearate, polyglyceryl-2 monoisostearate, polyglyceryl-3 isostearate, polyglyceryl-4 isostearate, polyglyceryl-6 monoisostearate, polyglyceryl-10 monoisostearate, polyglyceryl-2 monooleate, sorbitan isostearate, sorbitan monooleate, ethoxylated sorbitan monooleate containing 5 EO, and mixtures thereof.

**11.** Composition according to one of the preceding claims, **characterized in that** the hydrocarbon dispersant is selected from partial esters of polyglycerol and isostearic acid, partial esters of polyglycerol and oleic acid, partial esters of sorbitan and oleic acid, and mixtures thereof.

**12.** Composition according to one of the preceding claims, **characterized in that** the hydrocarbon dispersant is selected from polyglyceryl-3 diisostearate, polyglyceryl-2 monoisostearate, polyglyceryl-2 monooleate, sorbitan monooleate, and mixtures thereof.

**13.** Composition according to one of the preceding claims, **characterized in that** the hydrocarbon dispersant represents from 3 to 20% and more preferably from 5 to 15% of the total weight of the composition.

**14.** Composition according to one of the preceding claims, **characterized in that** the non-volatile hydrocarbon oil has

a molar mass ranging from 200 to 1 500 g/mol.

**15.** Composition according to one of the preceding claims, **characterized in that** the non-volatile hydrocarbon oil has a molar mass ranging from 220 to 500 g/mol.

**16.** Composition according to one of the preceding claims, **characterized in that** the non-volatile hydrocarbon oil has a molar mass ranging from 230 to 430 g/mol.

**17.** Composition according to one of the preceding claims, **characterized in that** the non-volatile hydrocarbon oil is selected from esters resulting from the reaction of a $C_2$ to $C_{30}$, more preferably $C_2$ to $C_{18}$, branched and saturated carboxylic acid with a $C_2$ to $C_{30}$, more preferably $C_2$ to $C_{20}$, alcohol or with a $C_2$ to $C_{20}$, more preferably $C_2$ to $C_8$, polyol, and mixtures thereof.

**18.** Composition according to one of the preceding claims, **characterized in that** the non-volatile hydrocarbon oil is selected from esters of neopentanoic acid, esters of isononanoic acid and mixtures thereof.

**19.** Composition according to one of the preceding claims, **characterized in that** the non-volatile hydrocarbon oil is selected from isodecyl neopentanoate, isotridecyl neopentanoate, isostearyl neopentanoate, octyldodecyl neopentanoate, isononyl isononanoate, octyl isononanoate, isodecyl isononanoate, isotridecyl isononanoate, isostearyl isononanoate, and mixtures thereof.

**20.** Composition according to one of the preceding claims, **characterized in that** the non-volatile hydrocarbon oil represents from 5 to 98%, preferably from 7 to 60%, more preferably from 10 to 50% and with particular preference from 10 to 30% of the total weight of the composition.

**21.** Composition according to one of the preceding claims, **characterized in that** the inert particulate phase comprises at least one absorbent or non-absorbent inert filler.

**22.** Composition according to the preceding claim, **characterized in that** the inert filler is selected from organic or inorganic, lamellar, spherical or oblong fillers.

**23.** Composition according to Claim 21 or 22, **characterized in that** the inert filler is selected from talc, mica, silica, kaolin, polyamide powders, poly-β-alanine powders and polyethylene powders, polytetrafluoroethylene powders, lauroyl lysine, starch, boron nitride, hollow polymeric microspheres, acrylic acid copolymers and silicone resin microbeads, precipitated calcium carbonate, dicalcium phosphate, magnesium carbonate and hydrocarbonate, hydroxyapatite, hollow silica microspheres, ceramic or glass microcapsules, metallic soaps derived from organic carboxylic acids having from 8 to 22 carbon atoms, preferably from 12 to 18 carbon atoms, for example zinc stearate, magnesium stearate or lithium stearate, zinc laurate, magnesium myristate, and mixtures thereof.

**24.** Composition according to one of the preceding claims, **characterized in that** the inert particulate phase represents from 0.1 to 30% of the total weight of the composition, more preferably from 2 to 25% and with particular preference from 3 to 20%.

**25.** Composition according to one of the preceding claims, **characterized in that** it comprises at least one colourant.

**26.** Composition according to the preceding claim, **characterized in that** the colourant comprises at least one pulverulent compound selected from pigments, nacres and mixtures thereof.

**27.** Composition according to Claim 25 or 26, **characterized in that** the colourant represents from 0.001 to 50%, preferably from 0.01 to 40% and more preferably from 0.05 to 30%, relative to the total weight of the composition.

**28.** Composition according to one of the preceding claims, **characterized in that** it comprises a non-volatile silicone compound.

**29.** Composition according to the preceding claim, **characterized in that** the non-volatile silicone compound is selected from compounds which are liquid at room temperature.

**30.** Composition according to Claim 28 or 29, **characterized in that** the non-volatile silicone compound has a viscosity

ranging from 5 to 100 000 cSt at 25°C, preferably ranging from 10 to 50 000 cSt and more preferably from 10 to 5 000 cSt.

**31.** Composition according to one of Claims 28 to 30, **characterized in that** the non-volatile silicone compound is selected from non-volatile polydimethylsiloxanes (PDMS); polydimethylsiloxanes containing alkyl, alkoxy or phenyl groups, pendently or at the silicone chain end, groups having from 2 to 24 carbon atoms; phenyltrimethicones, phenyldimethicones, phenyltrimethylsiloxydiphenylsiloxanes, diphenyldimethicones, diphenylmethyldiphenyltrisiloxanes and 2-phenylethyl trimethylsiloxysilicates; fluorinated silicones containing a fluorinated group pendently or at the silicone chain end, having from 1 to 12 carbon atoms, some or all of the hydrogens thereof being substituted by fluorine atoms; silicone resins; and mixtures thereof.

**32.** Composition according to one of Claims 28 to 31, **characterized in that** the non-volatile silicone compound represents from 0.5 to 90%, preferably from 5 to 60% and more preferably from 10 to 50% of the total weight of the composition.

**33.** Composition according to one of the preceding claims, **characterized in that** it further comprises at least one wax.

**34.** Composition according to the preceding claim, **characterized in that** the wax makes up from 0.01 to 50%, preferably from 2 to 40% and more preferably from 5 to 30% of the total weight of the composition.

**35.** Composition according to one of the preceding claims, **characterized in that** it further comprises at least one additive selected from antioxidants, preservatives, neutralizing agents, lipophilic gelling agents or liquid non-aqueous compounds, dispersants, active cosmetic substances, and mixtures thereof.

**36.** Composition according to one of the preceding claims, **characterized in that** it contains at least one active cosmetic substance selected from vitamins (A, E, C, $B_3$, F), provitamins, active soothing agents, plant extracts or essential oils, protective agents or restructuring agents, active freshness agents, emollients, moisturizers, active anti-wrinkle agents, essential fatty acids, and mixtures thereof.

**37.** Composition according to one of the preceding claims, **characterized in that** it is provided in the form of a cast or compacted product.

**38.** Makeup composition, in particular of the skin or lips, **characterized in that** it is in accordance with one of the preceding claims.

**39.** Composition according to one of the preceding claims, **characterized in that** it is provided in the form of a lipstick or lip gloss.

**40.** Cosmetic method of imparting to a film of anhydrous cosmetic composition properties of staying power, gloss, comfort and/or non-migration which consists in introducing into the said composition at least one non-volatile hydrocarbon oil, the said non-volatile oil being a branched and saturated ester, at least one inert particulate phase and at least one hydrocarbon dispersant which has solubility parameters $\delta_d$ and $\delta_a$ which meet the following conditions: $16.2 \leq \delta_d \leq 20\ (J/cm^3)^{1/2}$ and $9.1 \leq \delta_a \leq 20\ (J/cm^3)^{1/2}$, the said dispersant containing carbon and hydrogen atoms and one or more functional groups selected from hydroxyl, ester, ether, carboxylic and amide functions, the said hydrocarbon dispersant representing from 0.5 to 40% of the total weight of the composition, the said composition comprising less than 10% of volatile oil relative to the total weight of the composition.

**41.** Method according to the preceding claim, **characterized in that** the hydrocarbon dispersant is selected from:

- ether-modified fatty alcohols and in particular the addition products of ethylene oxide and/or propylene oxide with i) a linear or branched fatty alcohol or with ii) an alkylphenol,
- esters resulting from the reaction of at least one fatty acid with at least one addition product of ethylene oxide and glycerol or with at least one addition product of ethylene oxide and polyglycerol,
- esters resulting from the reaction of glycerol or polyglycerol with at least one addition product of ethylene oxide and a saturated or unsaturated fatty acid,
- partial esters resulting from the reaction of at least one saturated or unsaturated, linear or branched fatty acid, ricinoleic acid or 12-hydroxystearic acid with at least one polyol such as glycerol, polyglycerol, pentaerythritol, saccharide alcohols such as sorbitol, and especially esters of polyglycerol,

- esters resulting from the reaction of sorbitan with at least one saturated or unsaturated, linear or branched fatty acid,
- ether-modified sorbitan esters, and especially esters resulting iii) from the reaction of sorbitan with at least one addition product of ethylene oxide and a saturated or unsaturated fatty acid or iv) from the reaction of at least one saturated or unsaturated fatty acid with at least one addition product of ethylene oxide and sorbitan,
- addition products of ethylene oxide with castor oil and/or hydrogenated castor oil,
- trialkyl phosphates and alkyl mono-, di- and triphosphates,

and mixtures thereof.

**42.** Method according to Claim 40 or 41, **characterized in that** the non-volatile hydrocarbon oil has a molar mass ranging from 200 to 1 500 g/mol.

**43.** Method according to one of Claims 40 to 42, **characterized in that** the non-volatile hydrocarbon oil is selected from esters resulting from the reaction of a $C_2$ to $C_{30}$, more preferably $C_2$ to $C_{18}$, branched and saturated carboxylic acid with a $C_2$ to $C_{30}$, more preferably $C_2$ to $C_{20}$, alcohol or with a $C_2$ to $C_{20}$, more preferably $C_2$ to $C_8$, polyol, and mixtures thereof.

**44.** Method according to one of Claims 40 to 42, **characterized in that** the non-volatile hydrocarbon oil is selected from esters of neopentanoic acid, esters of isononanoic acid and mixtures thereof.

**45.** Method according to one of Claims 40 to 43, **characterized in that** the inert particulate phase is selected from talc, mica, silica, kaolin, polyamide powders, poly-β-alanine powders and polyethylene powders, polytetrafluoroethylene powders, lauroyl lysine, starch, boron nitride, hollow polymeric microspheres, acrylic acid copolymers and silicone resin microbeads, precipitated calcium carbonate, dicalcium phosphate, magnesium carbonate and hydrocarbonate, hydroxyapatite, hollow silica microspheres, ceramic or glass microcapsules, metallic soaps derived from organic carboxylic acids having from 8 to 22 carbon atoms, preferably from 12 to 18 carbon atoms, for example zinc stearate, magnesium stearate or lithium stearate, zinc laurate, magnesium myristate, and mixtures thereof.

**46.** Use of a combination of at least one non-volatile hydrocarbon oil, the said non-volatile oil being a branched and saturated ester, at least one inert particulate phase and at least one hydrocarbon dispersant containing carbon and hydrogen atoms and one or more functional groups selected from hydroxyl, ester, ether, carboxylic and amide functions, the said dispersant having solubility parameters $\delta_d$ and $\delta_a$ which meet the following conditions: $16.2 \leq \delta_d \leq 20$ $(j/cm^3)^{1/2}$ and $9.1 \leq \delta_a \leq 20$ $(j/cm^3)^{1/2}$, in an anhydrous composition which has good staying power and is glossy, comfortable and/or non-migrating, the said hydrocarbon dispersant representing from 0.5 to 40% of the total weight of the composition, the said composition comprising less than 10% of volatile oil relative to the total weight of the composition.

**47.** Use of a combination of at least one non-volatile hydrocarbon oil, the said non-volatile oil being a branched and saturated ester, at least one inert particulate phase and at least one hydrocarbon dispersant containing carbon and hydrogen atoms and one or more functional groups selected from hydroxyl, ester, ether, carboxylic and amide functions, the said dipersant having solubility parameters $\delta_d$ and $\delta_a$ which meet the following conditions: $16.2 \leq \delta_d \leq 20$ $(J/cm^3)^{1/2}$ and $9.1 \leq \delta_a \leq 20$ $(J/cm^3)^{1/2}$, in an anhydrous cosmetic composition, as an agent for imparting to the said composition properties of staying power, gloss, comfort and/or non-migration, the said hydrocarbon dispersant representing from 0.5 to 40% of the total weight of the composition, the said composition comprising less than 10% of volatile oil relative to the total weight of the composition.

**48.** Use according to Claim 46 or 47, **characterized in that** the hydrocarbon dispersant is selected from:

- ether-modified fatty alcohols and in particular the addition products of ethylene oxide and/or propylene oxide with i) a linear or branched fatty alcohol or with ii) an alkylphenol,
- esters resulting from the reaction of at least one fatty acid with at least one addition product of ethylene oxide and glycerol or with at least one addition product of ethylene oxide and polyglycerol,
- esters resulting from the reaction of glycerol or polyglycerol with at least one addition product of ethylene oxide and a saturated or unsaturated fatty acid,
- partial esters resulting from the reaction of at least one saturated or unsaturated, linear or branched fatty acid, ricinoleic acid or 12-hydroxystearic acid with at least one polyol such as glycerol, polyglycerol, pentaerythritol, saccharide alcohols such as sorbitol, and especially esters of polyglycerol,

- esters resulting from the reaction of sorbitan with at least one saturated or unsaturated, linear or branched fatty acid,
- ether-modified sorbitan esters, and especially esters resulting iii) from the reaction of sorbitan with at least one addition product of ethylene oxide and a saturated or unsaturated fatty acid or iv) from the reaction of at least one saturated or unsaturated fatty acid with at least one addition product of ethylene oxide and sorbitan,
- addition products of ethylene oxide with castor oil and/or hydrogenated castor oil,
- trialkyl phosphates and alkyl mono-, di- and triphosphates,

and mixtures thereof.

**49.** Use according to one of Claims 46 to 48, **characterized in that** the non-volatile hydrocarbon oil has a molar mass ranging from 200 to 1 500 g/mol.

**50.** Use according to one of Claims 46 to 49, **characterized in that** the non-volatile hydrocarbon oil is selected from esters resulting from the reaction of a $C_2$ to $C_{30}$, more preferably $C_2$ to $C_{18}$, branched and saturated carboxylic acid with a $C_2$ to $C_{30}$, more preferably $C_2$ to $C_{20}$, alcohol or with a $C_2$ to $C_{20}$, more preferably $C_2$ to $C_8$, polyol, and mixtures thereof.

**51.** Use according to one of Claims 46 to 50, **characterized in that** the non-volatile hydrocarbon oil is selected from esters of neopentanoic acid, esters of isononanoic acid and mixtures thereof.

**52.** Use according to one of Claims 46 to 51, **characterized in that** the inert particulate phase is selected from talc, mica, silica, kaolin, polyamide powders, poly-β-alanine powders and polyethylene powders, polytetrafluoroethylene powders, lauroyl lysine, starch, boron nitride, hollow polymeric microspheres, acrylic acid copolymers and silicone resin microbeads, precipitated calcium carbonate, dicalcium phosphate, magnesium carbonate and hydrocarbonate, hydroxyapatite, hollow silica microspheres, ceramic or glass microcapsules, metallic soaps derived from organic carboxylic acids having from 8 to 22 carbon atoms, preferably from 12 to 18 carbon atoms, for example zinc stearate, magnesium stearate or lithium stearate, zinc laurate, magnesium myristate, and mixtures thereof.

**Patentansprüche**

**1.** Wasserfreie Zusammensetzung zur Pflege oder zum Schminken von Keratinsubstanzen, die in einem kosmetisch akzeptablen Medium enthält:

- mindestens ein nichtflüchtiges Kohlenwasserstofföl, wobei das nichtflüchtige Öl ein verzweigter und gesättigter Ester ist,
- mindestens eine Partikelphase, die mindestens einen inerten Füllstoff enthält, und
- mindestens ein Dispergiermittel auf Kohlenwasserstoffbasis, das Kohlenstoffatome und Wasserstoffatome und eine oder mehrere Funktionen enthält, die unter den Funktionen Hydroxy, Ester, Ether, Carboxy und Amid ausgewählt sind, wobei das Dispergiermittel Solubilitätsparameter δd und δa aufweist, die die folgenden Bedingungen erfüllen: $16{,}2 \leq \delta_d \leq 20\ (J/cm^3)^{1/2}$ und $9{,}1 \leq \delta_a \leq 20\ (J/cm^3)^{1/2}$,

wobei das Dispergiermittel auf Kohlenwasserstoffbasis 0,5 bis 40 % des Gesamtgewichts der Zusammensetzung ausmacht und wobei die Zusammensetzung weniger als 10 % flüchtiges Öl, bezogen auf das Gesamtgewicht der Zusammensetzung, enthält.

**2.** Zusammensetzung nach Anspruch 1, **dadurch gekennzeichnet, dass** sie weniger als 5 % flüchtiges Öl, bezogen auf das Gesamtgewicht der Zusammensetzung, und besser weniger als 2 % enthält.

**3.** Zusammensetzung nach Anspruch 1, **dadurch gekennzeichnet, dass** das Dispergiermittel auf Kohlenwasserstoffbasis Solubilitätsparameter δd und δa aufweist, die die folgenden Bedingungen erfüllen: $16{,}3 \leq \delta_d \leq 19\ (J/cm^3)^{1/2}$ und $10 \leq \delta_a \leq 18{,}1\ (J/cm^3)^{1/2}$.

**4.** Zusammensetzung nach einem der vorhergehenden Ansprüche, **dadurch gekennzeichnet, dass** das Dispergiermittel auf Kohlenwasserstoffbasis Solubilitätsparameter δd und δa aufweist, die die folgenden Bedingungen erfüllen: $16{,}9 \leq \delta_d \leq 18\ (J/cm^3)^{1/2}$ und $13 \leq \delta_a \leq 14{,}5\ (J/cm^3)^{1/2}$.

**5.** Zusammensetzung nach einem der vorhergehenden Ansprüche, **dadurch gekennzeichnet, dass** das Dispergiermittel auf Kohlenwasserstoffbasis bei Raumtemperatur (25 °C) fluide ist und/oder bei 20 °C eine Brechzahl ≥ 1,45 aufweist.

**6.** Zusammensetzung nach einem der vorhergehenden Ansprüche, **dadurch gekennzeichnet, dass** das Dispergiermittel auf Kohlenwasserstoffbasis eine chemische Struktur aufweist, die mindestens eine polare Gruppe enthält, die unter den folgenden Gruppen ausgewählt ist: -COOH; -OH; Ethylenoxid: -(O-$CH_2$-$CH_2$-); Propylenoxid

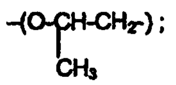

-$PO_4$ NHR; $NR_1R_2$, wobei $R_1$ und $R_2$ gegebenenfalls einen Ring bilden und eine geradkettige oder verzweigte Alkyl- oder Alkoxygruppe mit 1 bis 20 Kohlenstoffatomen bedeuten oder

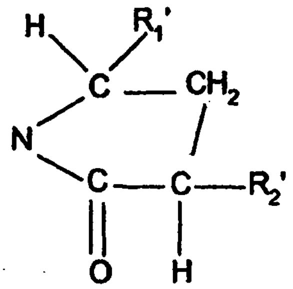

wobei $R_1$' und $R_2$' H oder eine geradkettige oder verzweigte Alkyl- oder Alkoxygruppe mit 1 bis 20 Kohlenstoffatomen bedeuten können.

**7.** Zusammensetzung nach einem der vorhergehenden Ansprüche, **dadurch gekennzeichnet, dass** das Dispergiermittel auf Kohlenwasserstoffbasis ausgewählt ist unter:

- Ether-modifizierten Fettalkoholen und insbesondere Additionsprodukten von Ethylenoxid und/oder Propylenoxid mit i) einem geradkettigen oder verzweigten Fettalkohol oder ii) einem Alkylphenol,
- Estern, die bei der Reaktion mindestens einer Fettsäure mit mindestens einem Additionsprodukt von Ethylenoxid und Glycerin oder mit mindestens einem Additionsprodukt von Ethylenoxid und Polyglycerin gebildet werden,
- Estern, die bei der Reaktion von Glycerin oder Polyglycerin mit mindestens einem Additionsprodukt von Ethylenoxid und einer gesättigten oder ungesättigten Fettsäure gebildet werden,
- partiellen Estern, die bei der Reaktion mindestens einer geradkettigen oder verzweigten, gesättigten oder ungesättigten Fettsäure, Ricinolsäure oder 12-Hydroxystearinsäure mit mindestens einem Polyol wie Glycerin, Polyglycerin, Pentaerythrit, Saccharidalkoholen wie Sorbit gebildet werden, und insbesondere Polyglycerylestern,
- Estern, die bei der Reaktion von Sorbitan mit mindestens einer geradkettigen oder verzweigten, gesättigten oder ungesättigten Fettsäure gebildet werden,
- Ether-modifizierten Sorbitanestern und insbesondere Estern, die iii) bei der Reaktion von Sorbitan mit mindestens einem Additionsprodukt von Ethylenoxid und einer gesättigten oder ungesättigten Fettsäure gebildet werden, oder iv) die bei der Reaktion von mindestens einer gesättigten oder ungesättigten Fettsäure mit mindestens einem Additionsprodukt von Ethylenoxid und Sorbitan gebildet werden,
- Additionsprodukten von Ethylenoxid und Ricinusöl und/oder hydriertem Ricinusöl,
- Trialkylphosphaten und alkylierten Mono-, Di- und Triphosphaten,

und deren Gemischen.

**8.** Zusammensetzung nach einem der vorhergehenden Ansprüche, **dadurch gekennzeichnet, dass** das Dispergiermittel auf Kohlenwasserstoffbasis unter den Monoestern, Diestern und Estern, die bei einer partiellen Veresterung gebildet werden, ausgewählt ist.

**9.** Zusammensetzung nach einem der vorhergehenden Ansprüche, **dadurch gekennzeichnet, dass** das Dispergiermittel auf Kohlenwasserstoffbasis ausgewählt ist unter:

- Additionsprodukten von 2 bis 30 mol Ethylenoxid und/oder 0 bis 5 mol Propylenoxid mit i) einem geradkettigen oder verzweigten $C_{8-40}$-Fettalkohol oder besser einem $C_{8-22}$-Fettalkohl oder mit ii) einem Alkylphenol,
- Estern, die bei der Reaktion mindestens einer $C_{8-40}$-Fettsäure und besser $C_{8-22}$-Fettsäure mit mindestens einem Additionsprodukt von 1 bis 30 mol Ethylenoxid und Glycerin oder mit mindestens einem Additionsprodukt von 1 bis 30 mol Ethylenoxid und Polyglycerin gebildet werden,
- Estern, die bei der Reaktion von Glycerin oder Polyglycerin mit mindestens einem Additionsprodukt von 2 bis 30 mol Ethylenoxid und einer gesättigten oder ungesättigten $C_{8-40-}$Fettsäure und besser $C_{8-22}$-Fettsäure gebildet werden,
- partiellen Estern, die bei der Reaktion mindestens einer geradkettigen oder verzweigten, gesättigten oder ungesättigten Fettsäure mit 8 bis 40 Kohlenstoffatomen und besser 8 bis 22 Kohlenstoffatomen, Ricinolsäure oder 12-Hydroxystearinsäure mit Glycerin, Polyglycerin, Pentaerythrit oder Sorbit gebildet werden,
- Estern, die bei der Reaktion von Sorbitan mit mindestens einer geradkettigen oder verzweigten, gesättigten oder ungesättigten Fettsäure mit 8 bis 40 Kohlenstoffatomen und besser 8 bis 22 Kohlenstoffatomen gebildet werden,
- Estern, die iii) bei der Reaktion von Sorbitan mit mindestens einem Additionsprodukt von 2 bis 30 mol Ethylenoxid und einer gesättigten oder ungesättigten $C_{8-40}$-Fettsäure und besser $C_{8-22}$-Fettsäure oder iv) bei der Umsetzung von mindestens einer gesättigten oder ungesättigten $C_{8-40}$-Fettsäure und besser $C_{8-22}$-Fettsäure mit mindestens einem Additionsprodukt von 2 bis 30 mol Ethylenoxid und Sorbitan gebildet werden,
- Additionsprodukten von 2 bis 60 mol Ethylenoxid und Ricinusöl und/oder hydriertem Ricinusöl,
- trialkylierten Phosphaten und alkylierten Mono-, Di- und Triphosphaten, und deren Gemischen.

**10.** Zusammensetzung nach einem der vorhergehenden Ansprüche, **dadurch gekennzeichnet, dass** das Dispergiermittel auf Kohlenwasserstoffbasis ausgewählt ist unter: mit 15 EO ethoxyliertem Myristylalkohol, mit 5 EO ethoxyliertem Diglycerylmonoisostearat, Triglyceryldiisostearat, Glycerylmonoisostearat, Diglycerylmonoisostearat, Triglycerylisostearat, Tetraglycerylisostearat, Hexaglycerylmonoisostearat, Decaglycerylmonoisostearat, Diglycerylmonooleat, Sorbitanisostearat, Sorbitanmonooleat, mit 5 EO ethoxyliertem Sorbitanmonooleat und deren Gemischen.

**11.** Zusammensetzung nach einem der vorhergehenden Ansprüche, **dadurch gekennzeichnet, dass** das Dispergiermittel auf Kohlenwasserstoffbasis unter den partiellen Estern von Polyglycerin und Isostearinsäure, partiellen Estern von Polyglycerin und Ölsäure, partiellen Estern von Sorbitan und Ölsäure und deren Gemischen ausgewählt ist.

**12.** Zusammensetzung nach einem der vorhergehenden Ansprüche, **dadurch gekennzeichnet, dass** das Dispergiermittel auf Kohlenwasserstoffbasis unter Triglyceryldiisostearat, Diglycerylmonoisostearat, Diglycerylmonooleat, Sorbitanmonooleat und deren Gemischen ausgewählt ist.

**13.** Zusammensetzung nach einem der vorhergehenden Ansprüche,
**dadurch gekennzeichnet, dass** das Dispergiermittel auf Kohlenwasserstoffbasis 3 bis 20 % und besser 5 bis 15 % des Gesamtgewichts der Zusammensetzung ausmacht.

**14.** Zusammensetzung nach einem der vorhergehenden Ansprüche, **dadurch gekennzeichnet, dass** das nichtflüchtige Kohlenwasserstofföl eine Molmasse von 200 bis 1500 g/mol hat.

**15.** Zusammensetzung nach einem der vorhergehenden Ansprüche, **dadurch gekennzeichnet, dass** das nichtflüchtige Kohlenwasserstofföl eine Molmasse von 220 bis 500 g/mol hat.

**16.** Zusammensetzung nach einem der vorhergehenden Ansprüche, **dadurch gekennzeichnet, dass** das nichtflüchtige Kohlenwasserstofföl eine Molmasse von 230 bis 430 g/mol hat.

**17.** Zusammensetzung nach einem der vorhergehenden Ansprüche, **dadurch gekennzeichnet, dass** das nichtflüchtige Kohlenwasserstofföl unter den Estern ausgewählt ist, die bei der Umsetzung einer verzweigten, gesättigten Carbonsäure mit 2 bis 30 Kohlenstoffatomen und besser 2 bis 18 Kohlenstoffatomen und einem Alkohol mit 2 bis

30 Kohlenstoffatomen und besser 2 bis 20 Kohlenstoffatomen oder einem Polyol mit 2 bis 20 Kohlenstoffatomen und besser 2 bis 8 Kohlenstoffatomen und deren Gemischen ausgewählt ist.

**18.** Zusammensetzung nach einem der vorhergehenden Ansprüche, **dadurch gekennzeichnet, dass** das nichtflüchtige Kohlenwasserstofföl unter den Estern von Neopentansäure, den Estern von Isononansäure und deren Gemischen ausgewählt ist.

**19.** Zusammensetzung nach einem der vorhergehenden Ansprüche, **dadurch gekennzeichnet, dass** das nichtflüchtige Kohlenwasserstofföl unter Isodecylneopentanoat, Isotridecylneopentonat, Isostearylneopentanoat, Octyldodecylneopentanoat, Isononylisononanoat, Octylisononanoat, Isodecylisononanoat, Isotridecylisononanoat, Isostearylisononanoat und deren Gemischen ausgewählt ist.

**20.** Zusammensetzung nach einem der vorhergehenden Ansprüche, **dadurch gekennzeichnet, dass** das nichtflüchtige Kohlenwasserstofföl 5 bis 98 %, vorzugsweise 7 bis 60 % und besser 10 bis 50 % und noch besser 10 bis 30 % des Gesamtgewichts der Zusammensetzung ausmacht.

**21.** Zusammensetzung nach einem der vorhergehenden Ansprüche, **dadurch gekennzeichnet, dass** die inerte Partikelphase mindestens einen absorbierenden oder nicht absorbierenden inerten Füllstoff enthält.

**22.** Zusammensetzung nach dem vorhergehenden Anspruch, **dadurch gekennzeichnet, dass** der inerte Füllstoff unter den anorganischen oder organischen, lamellaren, sphärischen oder länglichen Füllstoffen ausgewählt ist.

**23.** Zusammensetzung nach Anspruch 21 oder 22, **dadurch gekennzeichnet, dass** der inerte Füllstoff unter Talk, Glimmer, Kieselsäure, Kaolin, Polyamidpulvern, Poly-β-alaninpulvern, Polyethylenpulvern, Polytetrafluorethylenpulvern, Lauroyllysin, Stärke, Bornitrid, Polymermikrohohlkugeln, Copolymeren von Acrylsäure, Siliconharzmikrokugeln, gefälltem Calciumcarbonat, Bicalciumphosphat, Magnesiumcarbonat und Magnesiumhydrogencarbonat, Hydroxyapatit, Siliciumdioxidmikrohohlkugeln, Mikrokapseln aus Glas oder Keramik, Metallseifen, die von organischen Carbonsäuren mit 8 bis 22 Kohlenstoffatomen und vorzugsweise 12 bis 18 Kohlenstoffatomen abgeleitet sind, beispielsweise Zinkstearat, Magnesiumstearat oder Lithiumstearat, Zinklaurat und Magnesiummyristat, und deren Gemischen ausgewählt ist.

**24.** Zusammensetzung nach einem der vorhergehenden Ansprüche, **dadurch gekennzeichnet, dass** die inerte Partikelphase 0,1 bis 30 % des Gesamtgewichts der Zusammensetzung, besser 2 bis 25 % und noch besser 3 bis 20 % ausmacht.

**25.** Zusammensetzung nach einem der vorhergehenden Ansprüche, **dadurch gekennzeichnet, dass** sie mindestens ein Farbmittel enthält.

**26.** Zusammensetzung nach dem vorhergehenden Anspruch, **dadurch gekennzeichnet, dass** das Farbmittel mindestens eine pulverförmige Verbindung umfasst, die unter den Pigmenten, Perlglanzpigmenten und deren Gemischen ausgewählt ist.

**27.** Zusammensetzung nach Anspruch 25 oder 26, **dadurch gekennzeichnet, dass** das Farbmittel 0,001 bis 50 %, vorzugsweise 0,01 bis 40 % und besser 0,05 bis 30 %, bezogen auf das Gesamtgewicht der Zusammensetzung, ausmacht.

**28.** Zusammensetzung nach einem der vorhergehenden Ansprüche, **dadurch gekennzeichnet, dass** sie eine nichtflüchtige siliconierte Verbindung enthält.

**29.** Zusammensetzung nach dem vorhergehenden Anspruch, **dadurch gekennzeichnet, dass** die nichtflüchtige siliconierte Verbindung unter den bei Raumtemperatur flüssigen Verbindungen ausgewählt ist.

**30.** Zusammensetzung nach Anspruch 28 oder 29, **dadurch gekennzeichnet, dass** die nichtflüchtige siliconierte Verbindung bei 25 °C eine Viskosität von 5 bis 100.000 cSt, vorzugsweise 10 bis 50.000 cSt und besser 10 bis 5.000 cSt aufweist.

**31.** Zusammensetzung nach einem der Ansprüche 28 bis 30, **dadurch gekennzeichnet, dass** die nichtflüchtige siliconierte Verbindung unter den nichtflüchtigen Polydimethylsiloxanen (PDMS); Polydimethylsiloxanen, die Alkyl-,

Alkoxy- oder Phenylgruppen als Seitenketten oder am Ende der Siliconkette enthalten, wobei die Gruppen 2 bis 24 Kohlenstoffatome enthalten; Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilicaten; fluorierten Siliconen, die als Seitenkette oder am Ende der Siliconkette eine fluorierte Gruppe mit 1 bis 12 Kohlenstoffatomen enthalten, bei der alle oder ein Teil der Wasserstoffatome durch Fluoratome ersetzt sind; Siliconharzen; und deren Gemischen ausgewählt ist.

**32.** Zusammensetzung nach einem der Ansprüche 28 bis 31, **dadurch gekennzeichnet, dass** die nichtflüchtige siliconierte Verbindung 0,5 bis 90 %, vorzugsweise 5 bis 60 % und besser 10 bis 50 % des Gesamtgewichts der Zusammensetzung ausmacht.

**33.** Zusammensetzung nach einem der vorhergehenden Ansprüche, dass sie ferner mindestens ein Wachs enthält.

**34.** Zusammensetzung nach dem vorhergehenden Anspruch, **dadurch gekennzeichnet, dass** das Wachs 0,01 bis 50 %, vorzugsweise 2 bis 40 % und besser 5 bis 30 % des Gesamtgewichts der Zusammensetzung ausmacht.

**35.** Zusammensetzung nach einem der vorhergehenden Ansprüche, **dadurch gekennzeichnet, dass** sie ferner mindestens einen Zusatzstoff enthält, der unter den Antioxidantien, Konservierungsmitteln, Neutralisationsmitteln, lipophilen Gelbildnern oder nicht wässrigen flüssigen Verbindungen, Dispergiermitteln, kosmetischen Wirkstoffen und deren Gemischen ausgewählt ist.

**36.** Zusammensetzung nach einem der vorhergehenden Ansprüche, **dadurch gekennzeichnet, dass** sie mindestens einen kosmetischen Wirkstoff enthält, der unter den Vitaminen (A, E, C, $B_3$, F), Provitaminen, beruhigenden Stoffen, Pflanzenextrakten oder etherischen Ölen, Schutzmitteln oder restrukturierenden Wirkstoffen, erfrischenden Stoffen, Emollientien, Hydratisierungsmitteln, Antifaltenmitteln, essentiellen Fettsäuren und deren Gemischen ausgewählt ist.

**37.** Zusammensetzung nach einem der vorhergehenden Ansprüche, **dadurch gekennzeichnet, dass** sie als gegossenes oder kompaktiertes Produkt vorliegt.

**38.** Zusammensetzung zum Schminken, insbesondere der Haut oder der Lippen, **dadurch gekennzeichnet, dass** sie einem der vorhergehenden Ansprüche entspricht.

**39.** Zusammensetzung nach einem der vorhergehenden Ansprüche, **dadurch gekennzeichnet, dass** sie als Lippenstift oder Lippenglanz vorliegt.

**40.** Kosmetisches Verfahren, um einem Film einer wasserfreien kosmetischen Zusammensetzung die Eigenschaften Haftung, Glanz, Komfort und/oder Nichtmigration zu geben, das darin besteht, in die Zusammensetzung mindestens ein nichtflüchtiges Kohlenwasserstofföl, wobei das nichtflüchtige Öl ein verzweigter und gesättigter Ester ist, mindestens eine inerte Partikelphase und mindestens ein Dispergiermittel auf Kohlenwasserstoffbasis einzubringen, das Solubilitätsparameter δd und δa aufweist, die die folgenden Bedingungen erfüllen: $16{,}2 \leq \delta_d \leq 20\ (J/cm^3)^{1/2}$ und $9{,}1 \leq \delta_a \leq 20\ (J/cm^3)^{1/2}$, wobei das Dispergiermittel Kohlenstoffatome und Wasserstoffatome und eine oder mehrere Funktionen aufweist, die unter den Funktionen Hydroxy, Ester, Ether, Carboxy und Amid ausgewählt sind, wobei das Dispergiermittel auf Kohlenwasserstoffbasis 0,5 bis 40 % des Gesamtgewichts der Zusammensetzung ausmacht und wobei die Zusammensetzung weniger als 10 % flüchtiges Öl, bezogen auf das Gesamtgewicht der Zusammensetzung, enthält.

**41.** Verfahren nach dem vorhergehenden Anspruch, **dadurch gekennzeichnet, dass** das Dispergiermittel auf Kohlenwasserstoffbasis ausgewählt ist unter:

- Ether-modifizierten Fettalkoholen und insbesondere Additionsprodukten von Ethylenoxid und/oder Propylenoxid mit i) einem geradkettigen oder verzweigten Fettalkohol oder ii) einem Alkylphenol,
- Estern, die bei der Reaktion mindestens einer Fettsäure mit mindestens einem Additionsprodukt von Ethylenoxid und Glycerin oder mit mindestens einem Additionsprodukt von Ethylenoxid und Polyglycerin gebildet werden,
- Estern, die bei der Reaktion von Glycerin oder Polyglycerin mit mindestens einem Additionsprodukt von Ethylenoxid und einer gesättigten oder ungesättigten Fettsäure gebildet werden,
- partiellen Estern, die bei der Reaktion mindestens einer geradkettigen oder verzweigten, gesättigten oder

ungesättigten Fettsäure, Ricinolsäure oder 12-Hydroxystearinsäure mit mindestens einem Polyol wie Glycerin, Polyglycerin, Pentaerythrit, Saccharidalkoholen wie Sorbit gebildet werden, und insbesondere Polyglycerylestern,
- Estern, die bei der Reaktion von Sorbitan mit mindestens einer geradkettigen oder verzweigten, gesättigten oder ungesättigten Fettsäure gebildet werden,
- Ether-modifizierten Sorbitanestern und insbesondere Estern, die iii) bei der Reaktion von Sorbitan mit mindestens einem Additionsprodukt von Ethylenoxid und einer gesättigten oder ungesättigten Fettsäure gebildet werden oder iv) die bei der Reaktion von mindestens einer gesättigten oder ungesättigten Fettsäure mit mindestens einem Additionsprodukt von Ethylenoxid und Sorbitan gebildet werden,
- Additionsprodukten von Ethylenoxid und Ricinusöl und/oder hydriertem Ricinusöl,
- Trialkylphosphaten und alkylierten Mono-, Di- und Triphosphaten,

und deren Gemischen.

**42.** Verfahren nach Anspruch 40 oder 41, **dadurch gekennzeichnet, dass** das nichtflüchtige Kohlenwasserstofföl eine Molmasse von 200 bis 1500 g/mol aufweist.

**43.** Verfahren nach einem der Ansprüche 40 bis 42, **dadurch gekennzeichnet, dass** das nichtflüchtige Kohlenwasserstofföl unter den Estern, die bei der Umsetzung einer verzweigten, gesättigten Carbonsäure mit 2 bis 30 Kohlenstoffatomen und besser 2 bis 18 Kohlenstoffatomen und einem Alkohol mit 2 bis 30 Kohlenstoffatomen und besser 2 bis 20 Kohlenstoffatomen oder einem Polyol mit 2 bis 20 Kohlenstoffatomen und besser 2 bis 8 Kohlenstoffatomen gebildet werden, und deren Gemischen ausgewählt ist.

**44.** Verfahren nach einem der Ansprüche 40 bis 42, **dadurch gekennzeichnet, dass** das nichtflüchtige Kohlenwasserstofföl unter den Estern von Neopentansäure, den Estern von Isononansäure und deren Gemischen ausgewählt ist.

**45.** Verfahren nach einem der Ansprüche 40 bis 43, **dadurch gekennzeichnet, dass** die inerte Partikelphase unter Talk, Glimmer, Kieselsäure, Kaolin, Polyamidpulvern, Poly-β-alaninpulvern, Polyethylenpulvern, Polytetrafluorethylenpulvern, Lauroyllysin, Stärke, Bornitrid, Polymermikrohohlkugeln, Copolymeren von Acrylsäure, Siliconharzmikrokugeln, gefälltem Calciumcarbonat, Bicalciumphosphat, Magnesiumcarbonat und Magnesiumhydrogencarbonat, Hydroxyapatit, Siliciumdioxidmikrohohlkugeln, Mikrokapseln aus Glas oder Keramik, Metallseifen, die von organischen Carbonsäuren mit 8 bis 22 Kohlenstoffatomen und vorzugsweise 12 bis 18 Kohlenstoffatomen abgeleitet sind, beispielsweise Zinkstearat, Magnesiumstearat oder Lithiumstearat, Zinklaurat und Magnesiummyristat, und deren Gemischen ausgewählt ist.

**46.** Verwendung der Kombination aus mindestens einem nichtflüchtigen Kohlenwasserstofföl, wobei das nichtflüchtige Öl ein verzweigter und gesättigter Ester ist, mindestens einer inerten Partikelphase und mindestens einem Dispergiermittel auf Kohlenwasserstoffbasis, das Kohlenstoffatome und Wasserstoffatome und eine oder mehrere Funktionen aufweist, die unter den Funktionen Hydroxy, Ester, Ether, Carboxy und Amid ausgewählt sind, wobei das Dispergiermittel Solubilitätsparameter δd und δa aufweist, die die folgenden Bedingungen erfüllen: $16{,}2 \leq \delta_d \leq 20$ $(J/cm^3)^{1/2}$ und $9{,}1 \leq \delta_a \leq 20$ $(J/cm^3)^{1/2}$, in einer wasserfreien Zusammensetzung mit hoher Beständigkeit, die glänzt, angenehm ist und/oder nicht migriert, wobei das Dispergiermittel auf Kohlenwasserstoffbasis 0,5 bis 40 % des Gesamtgewichts der Zusammensetzung ausmacht und wobei die Zusammensetzung weniger als 10 % flüchtiges Öl, bezogen auf das Gesamtgewicht der Zusammensetzung, enthält.

**47.** Verwendung der Kombination aus mindestens einem nichtflüchtigen Kohlenwasserstofföl, wobei das nichtflüchtige Öl ein verzweigter und gesättigter Ester ist, mindestens einer inerten Partikelphase und mindestens einem Dispergiermittel auf Kohlenwasserstoffbasis, das Kohlenstoffatome und Wasserstoffatome und eine oder mehrere Funktionen aufweist, die unter den Funktionen Hydroxy, Ester, Ether, Carboxy und Amid ausgewählt sind, wobei das Dispergiermittel Solubilitätsparameter δd und δa aufweist, die die folgenden Bedingungen erfüllen: $16{,}2 \leq \delta_d \leq 20$ $(J/cm^3)^{1/2}$ und $9{,}1 \leq \delta_a \leq 20$ $(J/cm^3)^{1/2}$, in einer wasserfreien kosmetischen Zusammensetzung als Stoff, um der Zusammensetzung die Eigenschaften Beständigkeit, Glanz, Komfort und/ oder Nichtmigration zu geben, wobei das Dispergiermittel auf Kohlenwasserstoffbasis 0,5 bis 40 % des Gesamtgewichts der Zusammensetzung ausmacht und wobei die Zusammensetzung weniger als 10 % flüchtiges Öl, bezogen auf das Gesamtgewicht der Zusammensetzung, enthält.

**48.** Verwendung nach Anspruch 46 oder 47, **dadurch gekennzeichnet, dass** das Dispergiermittel auf Kohlenwasserstoffbasis ausgewählt ist unter:

- Ether-modifizierten Fettalkoholen und insbesondere Additionsprodukten von Ethylenoxid und/oder Propylenoxid mit i) einem geradkettigen oder verzweigten Fettalkohol oder ii) einem Alkylphenol,
- Estern, die bei der Reaktion mindestens einer Fettsäure mit mindestens einem Additionsprodukt von Ethylenoxid und Glycerin oder mit mindestens einem Additionsprodukt von Ethylenoxid und Polyglycerin gebildet werden,
- Estern, die bei der Reaktion von Glycerin oder Polyglycerin mit mindestens einem Additionsprodukt von Ethylenoxid und einer gesättigten oder ungesättigten Fettsäure gebildet werden,
- partiellen Estern, die bei der Reaktion mindestens einer geradkettigen oder verzweigten, gesättigten oder ungesättigten Fettsäure, Ricinolsäure oder 12-Hydroxystearinsäure mit mindestens einem Polyol wie Glycerin, Polyglycerin, Pentaerythrit, Saccharidalkoholen wie Sorbit gebildet werden, und insbesondere Polyglycerylestern,
- Estern, die bei der Reaktion von Sorbitan mit mindestens einer geradkettigen oder verzweigten, gesättigten oder ungesättigten Fettsäure gebildet werden,
- Ether-modifizierten Sorbitanestern und insbesondere Estern, die iii) bei der Reaktion von Sorbitan mit mindestens einem Additionsprodukt von Ethylenoxid und einer gesättigten oder ungesättigten Fettsäure gebildet werden, oder iv) die bei der Reaktion von mindestens einer gesättigten oder ungesättigten Fettsäure mit mindestens einem Additionsprodukt von Ethylenoxid und Sorbitan gebildet werden,
- Additionsprodukten von Ethylenoxid und Ricinusöl und/oder hydriertem Ricinusöl,
- Trialkylphosphaten und alkylierten Mono-, Di- und Triphosphaten,

und deren Gemischen.

**49.** Verwendung nach einem der Ansprüche 46 bis 48, **dadurch gekennzeichnet, dass** das nichtflüchtige Kohlenwasserstofföl eine Molmasse von 200 bis 1500 g/ mol hat.

**50.** Verwendung nach einem der Ansprüche 46 bis 49, **dadurch gekennzeichnet, dass** das nichtflüchtige Kohlenwasserstofföl unter den Estern, die bei der Umsetzung einer verzweigten, gesättigten Carbonsäure mit 2 bis 30 Kohlenstoffatomen und besser 2 bis 18 Kohlenstoffatomen und einem Alkohol mit 2 bis 30 Kohlenstoffatomen und besser 2 bis 20 Kohlenstoffatomen oder einem Polyol mit 2 bis 20 Kohlenstoffatomen und besser 2 bis 8 Kohlenstoffatomen gebildet werden, und deren Gemischen ausgewählt ist.

**51.** Verwendung nach einem der Ansprüche 46 bis 50, **dadurch gekennzeichnet, dass** das nichtflüchtige Kohlenwasserstofföl unter den Estern von Neopentansäure, den Estern von Isononansäure und deren Gemischen ausgewählt ist.

**52.** Verwendung nach einem der Ansprüche 46 bis 51, **dadurch gekennzeichnet, dass** die inerte Partikelphase unter Talk, Glimmer, Kieselsäure, Kaolin, Polyamidpulvern, Poly-β-alaninpulvern, Polyethylenpulvern, Polytetrafluorethylenpulvern, Lauroyllysin, Stärke, Bornitrid, Polymermikrohohlkugeln, Copolymeren von Acrylsäure, Siliconharzmikrokugeln, gefälltem Calciumcarbonat, Bicalciumphosphat, Magnesiumcarbonat und Magnesiumhydrogencarbonat, Hydroxyapatit, Siliciumdioxidmikrohohlkugeln, Mikrokapseln aus Glas oder Keramik, Metallseifen, die von organischen Carbonsäuren mit 8 bis 22 Kohlenstoffatomen und vorzugsweise 12 bis 18 Kohlenstoffatomen abgeleitet sind, beispielsweise Zinkstearat, Magnesiumstearat oder Lithiumstearat, Zinklaurat und Magnesiummyristat, und deren Gemischen ausgewählt ist.